# FASCICULE DE BREVET EUROPEEN

(11) **EP 1 017 385 B1**
(45) Date de publication et mention de la délivrance du brevet: **16.07.2003**
(21) Numéro de dépôt: 98925747.2
(22) Date de dépôt: 20.05.1998
(51) Int. Cl.: A61K 31/44, A61P 27/06, A61P 19/00, A61P 43/00

(54) **UTILISATION DE TETRAHYDROPYRIDINES 4-SUBSTITUEES POUR FABRIQUER DES MEDICAMENTS AGISSANT SUR LE TGF-BETA1**
VERWENDUNG VON 4-SUBSTITUIERTEN TETRAHYDROPYRIDINEN ZUR HERSTELLUNG EINES ARZNEIMITTELS MIT WIRKUNG AUF TGF-BETA-1
USE OF 4-SUBSTITUTED TETRAHYDROPYRIDINES FOR MAKING MEDICINES ACTING ON TGF-beta 1

(30) Priorité: 28.05.1997 FR 9706522
(43) Date de publication de la demande: 12.07.2000
(73) Titulaire: SANOFI-SYNTHELABO, 75013 Paris (FR)
(72) Inventeur: BONO, Françoise, F-31000 Toulouse (FR); FOURNIER, Jacqueline, F-31830 Plaisance du Touch (FR); HERBERT, Jean-Marc, F-31170 Tournefeuille (FR); LAMARCHE, Isabelle, F-31330 Larra (FR); GUZZI, Umberto, I-20148 Milan (IT)
(74) Mandataire: Thouret-Lemaitre, Elisabeth
(86) Numéro de dépôt international: FR9801000
(87) Numéro de publication internationale: WO98053821

(56) Documents cités:
- EP-A- 0 458 696
- EP-A- 0 498 718
- EP-A- 0 645 755
- WO-A-97/01536
- J.H. PREHN: "Protective effect of transforming growth factor-beta 1 on beta-amyloid neurotoxicity in rat hippocampal neurons" STN INTERNATIONAL, KARLSRUHE, FILE MEDLINE, AN=96226070, XP002054062 & MOLECULAR PHARMACOLOGY, vol. 49, no. 2, 1996, pages 319-328,
- J.P. TERRANOVA: "Administration of amyloid beta-peptides in the rat medial septum causes memory deficits: reversal by SR 57746A, a non-peptide neurotrophic compound" STN INTERNATIONAL, KARLSRUHE, FILE MEDLINE, AN=97011603, XP002054063 & NEUROSCIENCE LETTERS, vol. 213, no. 2, 1996, pages 79-82,
- J. MATHIEU: "Stress oxydatif et apoptose" STN INTERNATIONAL, KARLSRUHE, FILE MEDLINE, AN=97112057, XP002054064 cité dans la demande & ANNALES PHARMACEUTIQUES FRANÇAISES, vol. 54, no. 5, 1996, pages 193-201,

## Description

La présente invention concerne l'utilisation de certains dérivés de la 1,2,3,6-tétrahydropyridine, et de leurs sels et solvates pharmaceutiquement acceptables pour la préparation de médicaments aptes à augmenter les taux de TGF-β1 (de l'anglais "transforming growth factor-β1").

Le TGF-β1 est un peptide multifonctionnel et ubiquitaire constitué, dans sa forme active, de deux sous-unités identiques liées par une liaison disulfure. Comme illustré par P. Bedesse et V. Paradis (Journal of Hepatology, 1995, 22, 37-42), le TGF-β1 a été identifié comme facteur induisant la croissance cellulaire dans les fibroblastes transformés, mais plusieurs autres fonctions cellulaires ont été successivement découvertes.

La demande WO 93/09808 décrit l'utilisation du TGF-β1 pour le traitement des dommages du système nerveux central.

Les demandes WO 96/34881 et WO 94/17099 revendiquent de nouveaux peptides ayant une activité similaire à celle du TGF-β1 et utilisables pour le traitement de plusieurs pathologies.

Le TGF-β1 est par exemple impliqué dans le contrôle du cycle cellulaire, dans l'angiogénèse, dans la différentiation cellulaire, dans l'embryogénèse, dans la réparation tissulaire ainsi que dans l'apoptose.

Parmi ces activités, l'effet anti-apoptotique du TGF-β1 est très important à cause de ses implications pharmacologiques.

Par le terme apoptose ou mort cellulaire programmée, on indique l'ensemble des processus physiologiques liés à la mort cellulaire. Dans sa phase terminale, l'apoptose est caractérisée par une activation des endonucléases qui clivent l'ADN double brin dans des régions internucléosomales générant ainsi des mono et oligonucléosomes qui se complexent à des histones. On observe alors dans le cytoplasme des cellules apoptotiques un enrichissement en oligo et mononucléosomes liés à des histones.

Bien que ce phénomène, au contraire de la nécrose, soit physiologique, il peut être aussi provoqué par des stimulations pathologiques.

D.A. Carson et J. M. Ribeiro rapportent (The Lancet 1993, 341, 1251-1254) le rôle de l'apoptose dans certaines pathologies telles que l'immunodépression, les déficits immunitaires chez les patients atteints du SIDA, le viellissement cellulaire, les troubles dégénératifs. physiques, comme les radicaux libres et les radiations ionisantes sont entraînés par les effets pro-apoptotiques de ces agents.

Des produits régulateurs de l'apoptose ont été décrits dans la demande de brevet WO 96/21449. La formule générale inclut tant des inhibiteurs que des stimulateurs de l'apoptose, sans qu'il soit donné le moyen de distinguer les uns des autres.

Par ailleurs, la demande EP 0 458 696 décrit l'utilisation de la 1(2-naphtyléthyl)-4-(3-trifluorophényl)-1,2,3,6-tétrahydropyridine pour la préparation de médicaments destinés au traitement et/ou à la prophylaxie des maladies cérébrales et neuronales. La demande WO 97/01536 décrit des 1-phénylalkyl-1,2,3,6-tétrahydropyridines 4-substituées ayant une activité neurotrophique et neuroprotectrice. Il est également connu que des dérivés de 4-(3-trifluorométhylphényl)-1,2,3,4-tétrahydropyridiniques sont des capteurs de radicaux libres (demande EP 0 498 718).

Il a été maintenant trouvé que certaines tétrahydropyridines augmentent les taux circulants et cellulaires et extracellulaires de TGF-β1.
Ainsi, la présente invention a pour objet l'utilisation d'une 1,2,3,6-tétrahydropyridine 4-substituée de formule (I): 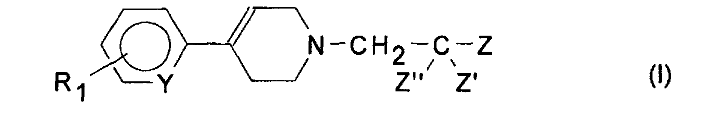 dans laquelle:
- R₁ représente un halogène ou un groupe CF₃, (C₁-C₄)alkyle ou (C₁-C₄)alcoxy;
- Y représente un atome d'azote ou un groupe CH;
- Z' et Z" représentent chacun l'hydrogène ou un groupe (C₁-C₃) alkyle, ou bien l'un représente l'hydrogène et l'autre un groupe hydroxy, ou encore les deux, ensemble, représentent un groupe oxo;
- Z représente
   ◆ un radical phényle;
   ◆ un radical phényle monosubstitué par un substituant X, X étant
      a) un groupe (C₁-C₆)alkyle; (C₁-C₆)alcoxy; (C₃-C₇)carboxyalkyle; (C₁-C₄)alcoxycarbonyl(C₁-C₆)alkyle; (C₃-C₇)carboxyalcoxy ou (C₁-C₄)-alcoxycarbonyl(C₁-C₆)alcoxy;
      b) un groupe choisi parmi un (C₃-C₇)cycloalkyle, (C₃-C₇)cycloalkyloxy, (C₃-C₇)cycloalkylméthyle, (C₃-C₇)cycloalkylamino et cyclohexényle, ledit groupe pouvant être substitué par un halogène, hydroxy, (C₁-C₄)alcoxy, carboxy, (C₁-C₄)alcoxycarbonyle, amino, mono- ou di-(C₁-C₄)alkylamino;
      c) un groupe choisi parmi un phényle, phénoxy, phénylamino, N-(C₁-C₃)alkylphénylamino, phénylméthyle, phényléthyle, phénylcarbonyle, phénylthio, phénylsulfonyle, phénylsulfinyle ou styryle, ledit groupe pouvant être mono- ou polysubstitué sur le groupe phényle par un halogène, CF₃, (C₁-C₄)alkyle, (C₁-C₄)alcoxy, cyano, amino, mono- ou di-(C₁-C₄)alkylamino, (C₁-C₄)acylamino, carboxy, (C₁-C₄)alcoxycarbonyle, aminocarbonyle, mono- ou
di-(C₁-C₄)alkylaminocarbonyle, amino(C₁-C₄)alkyle, hydroxy(C₁-C₄)alkyle ou halogéno(C₁-C₄)alkyle;
◆ un radical phényle disubstitué par un substituant R₂, R₂ étant un halogène ou un groupe hydroxy, méthyle, éthyle, (C₃-C₆)alkyle, (C₁-C₄)alcoxy ou trifluorométhyle et par un substituant X, X étant tel que défini ci-dessus;
◆ un radical 1-naphtyle ou 2-naphtyle;
◆ un radical 1-naphtyle ou 2-naphtyle, substitué dans les positions 5, 6, 7 et/ou 8 par un ou deux groupes hydroxyle, un ou deux groupes (C₁-C₄)alcoxy ou un groupe 6, 7-méthylènedioxy;
   - ou bien Z" est l'hydrogène et Z et Z' représentent, chacun indépendemment, un
   - groupe phényle non substitué ou mono-di ou trisubstitué;
      ou d'un de ses sels et solvates pharmaceutiquement acceptables, pour la préparation de compositions pharmaceutiques aptes à induire une augmentaion des taux circulants et cellulaires et extracellulaires de TGF-β1 et utiles pour le traitement des maladies choisies parmi les maladies à prions, le glaucome, l'ostéoporose et les fractures osseuses.

Selon un aspect avantageux, l'invention a pour objet l'utilisation du composé de formule (I) où Y est CH et R₁ est *o*- ou *m*-CF₃.

Selon un aspect préféré, Y est CH, R₁ est *o*- ou *m*-CF₃ et Z' et Z" sont l'hydrogène.

Selon un autre aspect préféré, Y est CH, R₁ est *o*- ou *m*-CF₃, Z' et Z" représentent un groupe oxo et Z est le 4-biphényle.

Selon un aspect avantageux ultérieur, l'invention a pour objet l'utilisation du composé de formule (I) où Y est CH, R₁ est *o*- ou *m*-CF₃, Z' et Z" sont l'hydrogène et Z représente un radical phényle monosubstitué par un substituant X, X étant a), b), c) ou de l'un de ses sels et solvates pharmaceutiquement acceptables.

Selon un autre aspect préféré, l'invention concerne l'utilisation du composé de formule (I) dans laquelle Y est CH, R₁ est *o*- ou *m*-CF₃, Z' et Z" sont l'hydrogène et Z représente soit un radical phényle monosubstitué par un groupe X', X' étant un phényle, non substitué ou substitué par 1 à 3 halogène, 1 à 3 CF₃, 1 à 3 (C₁-C₄)alkyle, 1 à 3 (C₁-C₄)alcoxy, 1 à 3 cyano, 1 à 3 amino, 1 à 3 mono- ou di-(C₁-C₄)alkylamino, 1 à 3 (C₁-C₄)acylamino, 1 à 3 carboxy, 1 à 3 (C₁-C₄)alcoxycarbonyle, 1 à 3 aminocarbonyle, 1 à 3 mono- ou di-(C₁-C₄)alkylaminocarbonyle, 1 à 3 amino(C₁-C₄)alkyle, 1 à 3 hydroxy(C₁-C₄)alkyle ou 1 à 3 halogéno(C₁-C₄)alkyle; soit un radical phényle disubstitué par un substituant R₂, R₂ étant un halogène ou un groupe hydroxy, méthyle, éthyle, (C₃-C₆)alkyle, (C₁-C₄)alcoxy ou trifluorométhyle et par un substituant X', X' étant tel que défini ci-dessus, ou de l'un de ses sels et solvates pharmaceutiquement acceptables.

Selon un autre aspect préféré, l'invention concerne l'utilisation du composé de formule (I) dans laquelle Y est CH, R₁ est *o*- ou *m*-CF₃, Z' et Z" sont l'hydrogène et Z est un groupe phényle substitué dans les positions 3 et 4 par un groupe (C₁-C₆) alkyle, ou de l'un de ses sels et solvates pharmaceutiquement acceptables.

Selon un autre aspect préféré, l'invention concerne l'utilisation du composé de formule (I) où Y est CH, R₁ est *o*- ou *m*-CF₃, Z" est l'hydrogène et Z et Z', identiques, représentent chacun un groupe phényle ; un groupe phényle substitué en position 2, 3 ou 4 par un atome de fluor, de chlore ou par un groupe méthyle, éthyle, *n*-propyle, *i*-propyle, *n*-butyle, *i*-butyle, *s*-butyle, *t*-butyle trifluorométhyle, cyano, méthoxy, méthylthio, méthylsulfonyle, éthoxy, éthylthio, éthylsulfonyle, (C₁-C₃)alcoxycarbonyle ou di(C₁-C₃)alkylaminocarbonyle; un groupe phényle disubstitué dans les positions 2, 4; 3, 4; 3, 5 ou 2, 6 par un atome de chlore, de fluor, ou par un groupe méthyle, éthyle, trifluorométhyle, cyano ou méthoxy; ou un groupe phényle trisubstitué dans les positions 3, 4, 5; 2, 4, 5 ou 2, 4, 6 par un atome de chlore, de fluor, ou par un groupe méthyle, éthyle, trifluorométhyle, cyano ou méthoxy, ou de l'un de ses sels et solvates pharmaceutiquement acceptables.

Selon un aspect particulièrement préféré, l'invention concerne l'utilisation du composé de formule (I) où Y est CH, R₁ est *m*-trifluorométhyle, Z' et Z" sont l'hydrogène et Z représente un groupe naphtyle, 6,7-diméthoxy-2-naphtyle ou 6,7-méthylènedioxy-2-naphtyle, ou de l'un de ses sels et solvates pharmaceutiquement acceptables.

Un composé particulièrement avantageux selon la présente invention peut être choisi parmi:
- 1-(2-napht-2-yléthyl)-4-(3-trifluorométhylphényl)-1,2,3,6-tétrahydropyridine;
- 1-[2-(6,7-diméthoxynapht-2-yl)éthyl)-4-(3-trifluorométhylphényl)-1,2,3,6-tétrahydropyridine;
- 1-[2-(6,7-méthylènedioxynapht-2-yl)éthyl)-4-(3-trifluorométhylphényl)-1,2,3,6-tétrahydropyridine;
- 1-[2-(4-isobutylphényl)-propyl]-4-(3-trifluorométhylphényl)-1,2,3,6-tétrahydropyridine;
- 1-[(2S)-2-(4-isobutylphényl)propyl]-4-(3-trifluorométhylphényl)-1,2,3,6-tétrahydropyridine;
- 1-[(2R)-2-(4-isobutylphényl)-propyl]-4-(3-trifluorométhylphényl)-1,2,3,6-tétrahydropyridine;
- 1-[2-(4-isobutylphényl)-éthyl]-4-(3-trifluorométhylphényl)-1,2,3,6-tétrahydropyridine;
- 1-[2-(4-tertbutylphényl)-éthyl]-4-(3-trifluorométhylphényl)-1,2,3,6-tétrahydropyridine;
- 1-[2-(4-isobutylphényl)-2-méthylpropyl]-4-(3-trifluorométhylphényl)-1,2,3,6-tétrahydropyridine;
- 1-[2-(4-isopropylphényl)éthyl]-4-(3-trifluorométhylphényl)-1,2,3,6-tétrahydropyridine;
- 1-[2-(3'-chloro-4-biphénylyl)éthyl]-4-(3-trifluorométhylphényl)-1,2,3,6-tétrahydropyridine;
- 1-[2-(2'-chlore-4-biphénylyl)-éthyl]-4-(3-trifluorométhylphényl)-1,2,3,6-tétrahydropyridine;
- 1-[2-(4'-chloro-4-biphénylyl)-éthyl]-4-(3-trifluorométhylphényl)- 1,2,3,6-tétrahydropyridine;
- 1-[2-(4'-fluoro-4-biphénylyl)-éthyl]-4-(3-trifluorométhylphényl)-1,2,3,6-tétrahydropyridine;
- 1-[2-(3'-trifluorométhyl-4-biphénylyl)-éthyl]-4-(3-trifluorométhylphényl)-1,2,3,6-tétrahydropyridine;
- 1-[2-(4-cyclohexylphényl)éthyl]-4-(3-trifluorométhylphényl)-1,2,3,6-tétrahydropyridine;
- 1-[-2-(4-biphénylyl)-2-éthyl]-4-(4-fluorophényl)-1,2,3,6-tétrahydropyridine;
- 1-[2-(4-biphénylyl)-2-méthylpropyl]-4-(3-trifluorométhylphényl)-1,2,3,6-tétrahydropyridine;
- 1-[2-(4-phénoxyphényl)-2-éthyl]-4-(3-trifluorométhylphényl)-1,2,3,6-tétrahydropyridine;
- 1-[2-(4-benzylphényl)-2-éthyl]-4-(3 -trifluorométhylphényl)-1,2,3,6-tétrahydropyridine;
- 1-[2-(4-n-butylphényl)éthyl]-4-(3-trifluorométhylphényl)-1,2,3,6-tétrahydropyridine;
- 1-[2-(4-biphénylyl)éthyl]-4-(3-trifluorométhylphényl)-1,2,3,6-tétrahydropyridine;
- 1-[2-(4-n-butoxyphényl)éthyl]-4-(3 -trifluorométhylphényl)-1,2,3,6-tétrahydropyridine;
- 1-[2-(3,4-diéthylphényl)éthyl]-4-(3-trifluorométhylphényl)-1,2,3,6-tétrahydropyridine;
- 1-[2-(3-méthyl-4-pentylphényl)éthyl]-4-(3-trifluorométhylphényl)-1,2,3,6-tétrahydropyridine;
- 1-[2-(4-méthyl-3-pentylphényl)éthyl]-4-(3-trifluorométhylphényl)-1,2,3,6-tétrahydropyridine;
- 1-[2-(3,4-diéthylphényl)éthyl]-4-(6-chloropyrid-2-yl)-1,2,3,6-tétrahydropyridine;
- 1-(2,2-diphényléthyl)-4-(3-trifluorométhylphényl)- 1,2,3,6-tétrahydropyridine;
- 1-[2,2-(4,4'-dichlorodiphényl)éthyl]-4-(3-trifluorométhylphényl)-1,2,3,6-tétrahydropyridine;
- 1-[2,2-(3,3'-bistrifluorométhyldiphényl)éthyl]-4-(3-trifluorométhylphényl)-1,2,3,6-tétrahydropyridine;
- 1-[2,2-(4,4'-diméthoxydiphényl)éthyl]-4-(3-trifluorométhylphényl)-1,2,3,6-tétrahydropyridine;
- 1-[2-(4-fluorophényl)-2-phényléthyl]-4-(3-trifluorométhylphényl)-1,2,3,6-tétrahydropyridine;
- 1-(3,3-diphénylpropyl)-4-(3-trifluorométhylphényl)-1,2,3,6-tétrahydropyridine;
- 1-[2,2-(4,4'-dichlorodiphényl)éthyl]-4-(6-chloropyrid-2-yl)-1,2,3,6-tétrahydropyridine;
- 1-[2-(3'-chlorobiphényl-4-yl)-2-oxoéthyl]-4-(3-trifluorométhylphényl)-1,2,3,6-tétrahydropyridine;
- 1-[2-(2'-chlorobiphényl-4-yl)-2-oxoéthyl]-4-(3-trifluorométhylphényl)-1,2,3,6-tétrahydropyridine;
- 1-[2-(4'-chlorobiphényl-4-yl)-2-oxoéthyl]-4-(3-trifluorométhylphényl)-1,2,3,6-tétrahydropyridine;
- 1-[2-(4-isobutylphényl)-2-oxoéthyl]-4-(3-trifluorométhylphényl)-1,2,3,6-tétrahydropyridine;
- 1-[2-(4-benzylphényl)-2-oxoéthyl]-4-(3-trifluorométhylphényl)-1,2,3,6-tétrahydropyridine;
- 1-[2-(4-cyclohexylphényl)-2-oxoéthyl]-4-(3-trifluorométhylphényl)-1,2,3,6-tétrahydropyridine;
- 1-[2-(4'-fluorobiphényl-4-yl)-2-oxoéthyl]-4-(3-trifluorométhylphényl)-1,2,3,6-tétrahydropyridine;
- 1-[2-(4-*n*-butylphényl)-2-oxoéthyl]-4-(3-trifluorométhylphényl)-1,2,3,6-tétrahydropyridine;
- 1-[2-(biphényl-4-yl)-2-oxoéthyl]-4-(3-trifluorométhylphényl)-1,2,3,6-tétrahydropyridine;
- 1-[2-(4-t-butylphényl)-2-oxoéthyl]-4-(3-trifluorométhylphényl)-1,2,3,6-tétrahydropyridine;
- 1-[2-(4'-trifluorométhylbiphényl-4-yl)-2-oxoéthyl]-4-(3-trifluorométhylphényl)-1,2,3,6-tétrahydropyridine;
- 1-[2-(2,3'-dichloro-4-biphénylyl)-éthyl]-4-(3-tri fluorométhylphényl)-1,2,3,6-tétrahydropyridine;
- 1-[2-(3-chloro-4-biphénylyl)-éthyl]-4-(3-tri fluorométhylphényl)-1,2,3,6-tétrahydropyridine;
- 1-[2-(3',5'-dichloro-4-biphénylyl)-éthyl]-4-(3-trifluorométhylphényl)-1,2,3,6-tétrahydropyridine;
- 1-[2-(2',4'-dichloro-4-biphénylyl)-éthyl]-4-(3-trifluorométhylphényl)-1,2,3,6-tétrahydropyridine;
- 1-[2-(2-chloro-4-biphénylyl)-éthyl]-4-(3-trifluorométhylphényl)-1,2,3,6-tétrahydropyridine;
- 1-[2-(3'-chloro-4-biphénylyl)-2-méthylpropyl]-4-(3-trifluorométhylphényl)-1,2,3,6-tétrahydropyridine;
- 1-[2-(2-fluoro-4-biphénylyl)-propyl]-4-(3-trifluorométhylphényl)-1,2,3,6-tétrahydropyridine;
- x1-[2-(4-méthoxy-3-biphénylyl)-éthyl]-4-(3-trifluorométhylphényl)-1,2,3,6-tétrahydropyridine;
- 1-[2-(4'-méthoxy-4-biphénylyl)-éthyl]-4-(3-trifluorométhylphényl)-1,2,3,6-tétrahydropyridine;
- 1-[2-(4'-hydroxy-4-biphénylyl)-éthyl]-4-(3-trifluorométhylphényl)-1,2,3,6-tétrahydropyridine;
- 1-[2-(4'-éthoxycarbonylbutoxy-4-biphénylyl)-éthyl]-4-(3-trifluorométhylphényl)-1,2,3,6-tétrahydropyridine;
- 1-[2-(3-biphénylyl)-éthyl]-4-(3-trifluorométhylphényl)-1,2,3,6-tétrahydropyridine;
- 1-[2-(3'chloro-4'-fluoro-4-biphénylyl)-éthyl]-4-(3-trifluorométhylphényl)-1,2,3,6-tétrahydropyridine;
- 1-[2-(2'-trifluorométhyl-4-biphénylyl)-éthyl]-4-(3-trifluorométhylphényl)-1,2,3,6-tétrahydropyridine;
- 1-[2-(biphényl-4-yl)éthyl]-4-(2-trifluorométhylphényl)-1,2,3,6-tétrahydropyridine;
- 1-[2-(4-cyclohexénylphényl)éthyl]-4-(3-trifluorométhylphényl)-1,2,3,6-tétrahydropyridine;
- 1-[2-(3,4-diisobutylphényl)-éthyl]-4-(3-trifluorométhylphényl)-1,2,3,6-tétrahydropyridine;
- 1-[2-(3,4-dipropylphényl)-éthyl]-4-(3-trifluorométhylphényl)-1,2,3,6-tétrahydropyridine;
- 1-[2-(4-cyclohexylphényl)-éthyl]-4-(6-chloropyrid-2-yl)-1,2,3,6-tétrahydropyridine;
- 1-[2-(4-isobutylphényl)-propyl]-4-(6-chloropyrid-2-yl)-1,2,3,6-tétrahydropyridine;
- 1-[2-(2'-trifluorométhylbiphényl-4-yl)-2-oxoéthyl]-4-(3-trifluorométhylphényl)-1,2,3,6-tétrahydropyridine;
- 1-[2-(3'-trifluorométhylbiphényl-4-yl)-2-oxoéthyl]-4-(3-trifluorométhylphényl)-1,2,3,6-tétrahydropyridine;
   et leurs sels et solvates pharmaceutiquement acceptables.

La 1-(2-napht-2-yléthyl)-4-(3-trifluorométhylphényl)-1,2,3,6-tétrahydropyridine, connue par son code de laboratoire SR 57746 et ses sels et solvates pharmaceutiquement acceptables, notamment son chlorhydrate (SR 57746A), sont des composés particulièrement préférés pour l'utilisation selon la présente invention.

Certains des composés de formule (I) sont des produits nouveaux. Ainsi selon un autre de ses aspects la présente invention concerne un composé de formule (I) choisi parmi:
la 1-[2-(6,7-méthylènedioxynapht-2-yl)éthyl]-4-(3-trifluorométhylphényl)-1,2,3,6-tétrahydropyridine
la 1-[2-(4-cyclohexènylphényl)éthyl]-4-(3-trifluorométhylphényl)-1,2,3,6-tétrahydropyridine
la 1-[2-(biphényl-4-yl)éthyl]-4-(2-trifluorométhylphényl)-1,2,3,6-tétrahydropyridine
et leurs sels et solvates pharmaceutiquement acceptables.

Les sels avec des bases pharmaceutiquement acceptables sont par exemple ceux avec les métaux alcalins ou alcalino-terreux, tels que le sodium, le potassium, le calcium, le magnésium et ceux avec les bases organiques, telles que les amines, les acides aminés basiques (la lysine, l'arginine, l'histidine), le trométamol, la N-méthylglutamine, etc.

Les sels avec des acides pharmaceutiquement acceptables sont par exemple, ceux avec les acides minéraux, tels que le chlorhydrate, le bromhydrate, le borate, le phosphate, le sulfate, l'hydrogénusulfate, l'hydrogénophosphate et ceux avec les acides organiques, tels que le citrate, le benzoate, l'ascorbate, le méthylsulfate, les naphtalène-2-sulfonate, le picrate, le fumarate, le maléate, le malonate, l'oxalate, le succinate, l'acétate, le tartrate, le mésylate, le tosylate, l'iséthionate, l'α-cétoglutarate, l'α-glycérophosphate, le glucose-1-phosphate, etc.

Les composés de formule (I) où Z' et Z" sont l'hydrogène ou un groupe (C₁-C₃)alkyle sont préparés comme décrit dans WO 97/01536.

Les composés de formule (I) où l'un d'entre Z' et Z" est l'hydrogène et l'autre est un hydroxyle ainsi que les composés où Z' et Z" ensemble représentent un groupe oxo peuvent être préparés comme décrit dans WO 93/11107.

Les composés de formule (I) où Z" est l'hydrogène et Z' et Z représentent chacun indépendamment un groupe phényle non substitué, mono, di-, ou trisubstitué sont préparés selon le procédé suivant:
(a) on fait réagir une aryl-1,2,3,6-tétrahydropyridine de formule (II) 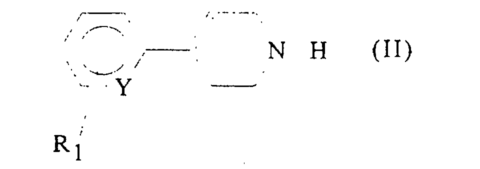 dans laquelle Y et R₁ sont tels que définis ci-dessus, avec un acide de formule (III) 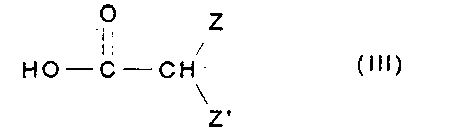 dans laquelle Z et Z sont tels que définis ci-dessus, ou l'un de ses dérivés fonctionnels,
(b) on réduit le carbonyle intermédiaire de formule (IV) 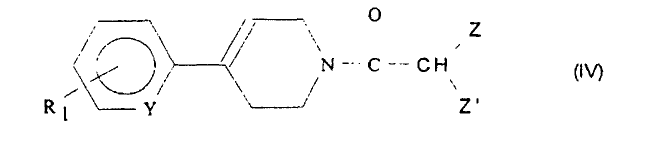
(c) on isole le composé de formule (I) ainsi obtenu et, éventuellement on le transforme en l'un de ses sels ou solvates.

La réaction de l'étape (a) peut être convenablement effectuée dans un solvant organique à une température comprise entre -10°C et la température de reflux du mélange réactionnel; de préférence la réaction est effectuée à basse température.

Comme solvant de réaction, on utilise de préférence un solvant halogéné tel que le chlorure de méthylène, le dichloroéthane, le 1,1,1-trichloroéthane, le chloroforme et similaires ou un alcool, tel que le méthanol ou l'éthanol, mais d'autres solvants organiques compatibles avec les réactifs employés, par exemple le dioxane, le tétrahydrofuranne ou un hydrocarbure tel que l'hexane, peuvent être également employés.

La réaction peut être convenablement effectuée en présence d'un accepteur de protons, par exemple d'un carbonate alcalin ou d'une amine tertiaire. Comme dérivé fonctionnel approprié de l'acide de formule (III), on peut utiliser l'acide libre, éventuellement activé (par exemple avec le BOP), l'anhydride, un anhydride mixte, un ester activé ou un halogénure d'acide, de préférence le chlorure ou bromure. Parmi les esters activés, l'ester de *p*-nitrophényle est particulièrement préféré, mais les esters de méthoxyphényle, de trityle, de benzhydryle et similaires sont également convenables.

La réduction de l'étape (b) peut être convenablement réalisée par des agents de réduction appropriés tels que les hydrures d'aluminium ou un hydrure complexe de lithium et d'aluminium dans un solvant organique inerte à une température comprise entre 0°C et la température de reflux du mélange réactionnel, selon les techniques usuelles.

Par "solvant organique inerte" on entend un solvant qui n'interfère pas avec la réaction. De tels solvants sont par exemple les éthers, tel que l'éther diéthylique, le tétrahydrofuranne, le dioxane ou le 1,2-diméthoxyéthane.

Le composé de formule (I) obtenu est isolé selon les techniques usuelles et éventuellement transformé en l'un de ses sels d'addition d'acides ou, lorsqu'un groupe acide est présent, le caractère amphotère du composé permet la séparation des sels soit avec des acides soit avec des bases.

Les amines de départ de formule (II) où Y est CH sont des composés connus ou bien elles peuvent être préparées selon des procédés analogues à ceux utilisés pour préparer les composés connus.

Les amines de départ de formule (II) où Y est N peuvent être préparées par réaction de la 2-halogénopyridine appropriée de formule (p) 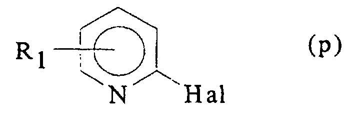 dans laquelle R₁ est tel que défini ci-dessus et Hal est un atome d'halogène, avec une 1,2,3,6-tétrahydropyridine de formule (q) 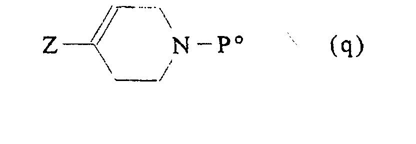 dans laquelle P° représente un groupe protecteur tel que par exemple le groupe benzyle et Z représente un substituant qui permet la substitution nucléophile de l'halogène de la pyridine. De tels substituants sont par exemple les trialkylstannanes, comme le tributylstannane ou les composés de Grignard.

On déprotège ensuite la 1,2,3,6-tétrahydropyridine par clivage du groupe protecteur dans des conditions convenables.

Les acides de formule (III) peuvent être préparés, selon la réaction de Wittig, par
a) réaction entre une benzophénone appropriée de formule (r) 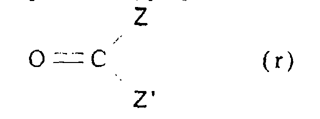
dans laquelle Z et Z' sont tels que définis ci-dessus avec l'iodure de triméthylsulfoxonium/BF₃-Et₂O et oxydation de l'aldéhyde intermédiaire de formule (w) 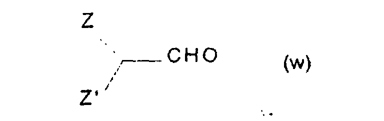 selon la méthode décrite dans J. Am. Chem. Soc., 1990, 112(18):6690-6695 pour obtenir l'acide correspondant.

Selon un autre mode opératoire, les composés de formule (I) où Z" est l'hydrogène peuvent également être préparés par réaction entre une aryl-1,2,3,6-tétrahydropyridine de formule (II) 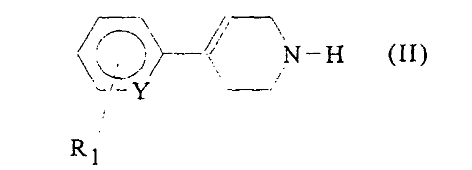 dans laquelle R₁ et Y sont tels que définis ci-dessus, et un aldéhyde de formule (w) ci-dessus en présence d'un agent de réduction tel que le cyanoborohydrure de sodium, selon les techniques connues.

Les composés de formule (I), dans laquelle R₁ est m-trifluorométhyle, Y est CH, Z' et Z" sont l'hydrogène et Z est un groupe naphtyle substitué par un ou deux groupes alcoxy ou par un groupe méthylènedioxy sont préparés comme décrit dans EP 0 458 697.

La 1-(2-napht-2-yléthyl)-4-(3-trifluorométhylphényl)-1,2,3,6-tétrahydropyridine et ses sels et solvates pharmaceutiquement acceptables, notamment le chlorhydrate, peuvent être préparés selon EP 0 101 381.

Une méthode avantageuse prévoit la réaction entre le 2-(2-bromoéthyl)naphtalène et la 4-(3-trifluorométhylphényl)-1,2,3,6-tétrahydropyridine et l'isolement de préférence du chlorhydrate de 1-(2-napht-2-yléthyl)-4-(3-trifluorométhylphényl)-1,2,3,6-tétrahydropyridine (SR 57746A) qui est par la suite cristallisé dans un melange éthanol/eau par chauffage et refroidissement à 5°C avec une rampe de refroidissement de 10°C/heure et une vitesse d'agitation de 400 tr/minute, de façon à obtenir un melange de deux formes cristallines dans un rapport d'environ 66/34.

Le chlorhydrate de 1-(2-napht-2-yléthyl)-4-(3-trifluorométhylphényl)-1,2,3,6-tétrahydropyridine est utilisé de préférence sous forme microparticulaire, par exemple sous une forme essentiellement amorphe obtenue par atomisation ou sous une forme microcristalline obtenue par micronisation.

L'effet des composés de formule (I) sur l'augmentation des taux de TGF-β1 a été évalué à l'aide d'essais sur des cellules musculaires lisses, ainsi que sur les taux sanguins et les diaphragmes chez le rat après administration de composés représentatifs de l'invention.

Sur les cellules musculaires lisses, on a dosé aussi bien le TGF-β1 latent que le TGF-β1 activé par incubation avec l'acide chlorhydrique.

Dans ces essais, des composés réprésentatifs de formule (I) ont montré une augmentation des taux de TGF-β1.

L'activité anti-apoptotique a été mesurée sur les mêmes cellules vis-à-vis de l'activité pro-apoptotique d'une déprivation en sérum ou après l'ajout de composés toxiques tels que la vincristine ou de facteurs de croissance tels que le facteur de croissance des nerfs (indiqué comme "nerve growth factor" : NGF) à l'aide d'un kit de dosage ELISA (de l'anglais "enzyme-linked immunosorbent assay) spécifique detectant la presence d'oligonucléosomes dont la présence à l'intérieur des cellules est un marqueur spécifique de la mort cellulaire programmée (apoptose), selon la méthode décrite par Del Bino G. et al., (Experimental Cell Research, 193, 27, 1991 et 195, 485, 1991) ou Darzynkiewicz A et al., (Cytometry, 13, 795, 1992).

Dans les trois cas, des composés représentatifs de l'invention, notamment:
- 1-[2-(3,4-diéthylphényl)éthyl]-4-(3-trifluorométhylphényl)-1,2,3,6-tétrahydropyridine (composé A);
- 1-[2-(biphényl-4-yl)-2-oxoéthyl]-4-(3-trifluorométhylphényl)-1,2,3,6-tétrahydropyridine (composé B);
- 1-[2-(biphényl-4-yl)éthyl]-4-(3-trifluorométhylphényl)-1,2,3,6-tétrahydropyridine (composé C);
- 1-[2-(6,7-méthylènedioxynapht-2-yl)éthyl]-4-(3-trifluorométhylphényl)-1,2,3,6-tétrahydropyridine (composé D);
- 1-[2-(4-cyclohexénylphényl)éthyl]-4-(3-trifluorométhylphényl)-1,2,3,6-tétrahydropyridine (composé E)
- 1-[2-(biphényl-4-yl)éthyl]-4-(2-trifluorométhylphényl)-1,2,3,6-tétrahydropyridine (composé F); et
- 1-(2-napht-2-yléthyl)-4-(3-trifluorométhylphényl)-1,2,3,6-tétrahydropyridine (SR 57746)
   et leurs sels pharmaceutiquement acceptables, inhibent, en fonction du temps et de la dose, l'effet pro-apoptotique induit par la déprivation en sérum ou bien par l'ajout de NGF ou de vincristine.

Ainsi, selon un aspect ultérieur, la présente invention concerne l'utilisation des tétrahydropyridines de formule (I), d'un des composés avantageux ou préférés cités ci-dessus, ou de leurs sels et solvates pharmaceutiquement acceptables pour la préparation d'un médicament destiné au traitement des maladies pouvant être soignées par une augmentation des taux de TGF-β1.
De telles pathologies sont par exemple les maladies, le glaucome, l'ostéoporose et les fractures osseuses.

Selon un aspect particulièrement avantageux, la présente invention concerne l'utilisation des tétrahydropyridines de formule (I), d'un des composés avantageux ou préférés cités ci-dessus, ou de leurs sels et solvates pharmaceutiquement acceptables pour la préparation de médicaments aptes à inhiber l'apoptose.

C'est grâce à cette activité anti-apoptotique que les composés de la présente invention peuvent être utilisés pour la préparation de médicaments destinés au traitement des maladies à prions.

Selon un aspect ultérieur, l'invention concerne une méthode pour augmenter les taux circulants et cellulaires et extracellulaires de TGF-β1.

Selon un autre de ses aspects, la présente invention concerne une méthode pour inhiber l'apoptose, qui comprend l'administration à un mammifère en ayant besoin d'une dose efficace d'un composé de formule (I), d'un des composés avantageux ou préférés cités ci-dessus, ou d'un de leurs sels et solvates pharmaceutiquement acceptables, avantageusement le SR 57746, ou un de ses sels et solvates pharmaceutiquement acceptables.

Selon un aspect préféré, le SR 57746 et ses sels et solvates pharmaceutiquement acceptables sont administrés sous forme microparticulaire, de préférence sous forme microparticulaire du chlorhydrate.

Les composés de formule (I), l'un des composés avantageux ou préférés cités ci-dessus ou leurs sels et solvates pharmaceutiquement acceptables sont de préférence administrés par voie orale.

La quantité de principe actif à administrer dépend du degré d'avancement de la maladie ainsi que de l'âge et du poids du patient. Néanmoins, les doses unitaires comprennent généralement de 0,25 à 700 mg, avantageusement de 0,5 à 300 mg, de préférence de 1 à 150 mg, par exemple entre 2 et 50 mg de principe actif. Ces doses unitaires sont administrées normalement une ou plusieurs fois par jour, de préférence une à trois fois par jour, la dose globale chez l'homme étant variable entre 0,5 et 1400 mg par jour, par exemple de 1 à 900 mg par jour, avantageusement de 2 à 500 mg par jour, plus convenablement de 2 à 200 mg par jour. Lorsque le principe actif administré est par exemple le SR 57746, la dose unitaire comprend généralement de 0,5 à 10 mg, avantageusement de 1 à 5, de préférence de 1 à 3 mg, par exemple 1 - 1,5 - 2 - 2,5 - 3 mg de principe actif. Ces doses unitaires sont administrées normalement une ou plusieurs fois par jour, de préférence une à trois fois par jour, la dose globale chez l'homme étant variable entre 0,5 et 50 mg par jour, par exemple de 1 à 20 mg par jour, avantageusement de 2 à 10 mg par jour.

Les doses et quantités ci-dessus se réfèrent aux composés de formule (I) ou à l'un des composés avantageux ou préférés cités ci-dessus, sous forme non-salifiée.

Dans les compositions pharmaceutiques de la présente invention pour l'administration orale, le principe actif peut être administré sous formes unitaires d'administration, en mélange avec des supports pharmaceutiques classiques, aux mammifères, aux animaux et aux êtres humains pour le traitement des affections susmentionnées. Les formes unitaires d'administration appropriées comprennent par exemple les comprimés éventuellement sécables, les gélules, les poudres, les granules et les solutions ou suspensions orales.

Lorsque l'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'elixir peut contenir l'ingrédient actif conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Le principe actif peut être formulé également sous forme de microcapsules, éventuellement avec un ou plusieurs supports ou additifs.

Dans les compositions pharmaceutiques selon la présente invention, le principe actif peut être aussi sous forme de complexe d'inclusion dans des cyclodextrines, leurs éthers ou leurs esters. Les PREPARATIONS et les EXEMPLES ci-dessous illustrent mieux l'invention.

### PREPARATION 1

40 000 cellules musculaires lisses isolées de l'aorte humaine (fournisseur : CLONETICS) sont placées dans une boîte de 35 mm dans un milieu contenant 2 ml de DMEM ( Milieu de Eagle modifié par Dulbecco contenant 4,5 g/l de glucose, 3,7 g/l de NaHCO₃ et ne contenant pas de L-glutamine ni de Na-pyruvate). On ajoute 20% v/v de sérum foetal de veau décomplémenté 30 min. à 80°C, de la L-glutamine 4 mM, 50 U/ml de pénicilline et 50 µg/ml de streptomycine. On laisse les cellules dans ce milieu pendant une période de croissance de trois jours avant de les soumettre aux tests selon les exemples rapportés plus loin.

### PREPARATION 2

On prépare des boîtes contenant des cellules comme décrit dans la Préparation 1.On induit l'apoptose en remplaçant le milieu décrit dans la Préparation 1 par le même milieu ne contenant que 0,2% de sérum foetal de veau. On mesure l'effet des composés de l'invention sur les taux de TGF-β1 latent et activé dans les milieux extracellulaires après 24 heures de contact avec les cellules en comparaison avec les témoins ( 0,2 % de sérum foetal de veau et 20% de sérum foetal de veau). Le TGF-β1 activé est dosé directement dans les surnageants de culture, en revanche le TGF-β1 latent est dosé après activation. Pour l'activation 0,5 ml de surnageant de culture sont incubés en présence de 0,1 ml de HCl 1N 10 min. à température ambiante. Le mélange est ensuite neutralisé avec 0,1 ml de tampon Hepes 0,5 M contenant du NaOH 1,2 N. Les dosages du TGF-β1 se font à l'aide d'un test ELISA spécifique.

### PREPARATION 3

5 rats de race Sprague Dawley (Iffa Credo, France) de 280g environ ont été traités quotidiennement pendant 3 jours par le composé à tester, administré par voie orale. 24 heures après le dernier gavage, les rats sont anesthésiés. On prélève le sang par l'aorte abdominale sur EDTA, les échantillons sont centrifugés et les surnageants (plasma riche en plaquettes) sont congelés. Les diaphragmes sont également prélevés, rincés plusieurs fois dans le PBS ( de l'anglais Phosphate buffered saline) froid et centrifugés. Après une ultracentrifugation ultérieure les culots sont repris par du PBS et congelés. Les dosages de TGF-β1 latent et activé dans les plasmas et les broyats des diaphragmes sont réalisés par la technique décrite dans la PREPARATION 2. On enregistre l'augmentation des taux de TGF-β1 latent circulant chez les rats traités par les composés de l'invention par rapport aux rats témoins. On enregistre aussi l'augmentation des taux de TGF-β1 activé dans les diaphragmes des rats traités avec les composés de l'invention.

### PREPARATION 4

Les boîtes contenant les cellules sont préparées comme dans la PREPARATION 1. On induit l'apoptose par trois méthodes differents:
a) en remplaçant le milieu de la PREPARATION 1 par le même milieu ne contenant que 0,2% de sérum foetal de veau.
b) en ajoutant au milieu décrit dans la PREPARATION 1 des doses croissantes de NGF (0,01 ng/ml à 100 ng/ml)
c) en ajoutant au milieu décrit dans la PREPARATION 1 des doses croissantes de Vincristine (0,1 pg/ml à 10 ng/ml)

Par un test ELISA de dosage des mono- et oligonucléosomes associés aux histones cytoplasmiques après lavage et lyse cellulaire, les effets des composés de l'invention sur l'apoptose sont mesurés après 24 heures de contact avec les cellules en comparaison avec les taux d'apoptose obtenus en absence de produits (taux maximum d'apoptose) ou en présence de 20% de sérum foetal de veau (taux minimum d'apoptose).

### EXEMPLE 1

### 1-(2,2-diphényléthyl)-4-(3-trifluorométhylphényl)-1,2,3,6-tétrahydropyridine et son chlorhydrate

### 1a/ 1-(α,α-diphénylacétyl)-4-(3-trifluorométhylphényl)-1,2,3,6-tétrahydropyridine

A un mélange de 8 g (0,035 mole) de 4-(3-trifluorométhylphényl)-1,2,3,6-tétrahydropyridine, 50 ml de chlorure de méthylène et 4,96 ml de triéthylamine on ajoute goutte à goutte à la température de 0/+5°C, 8 g de chlorure de α,α-diphénylacétyle dans 50 ml de chlorure de méthylène. On agite pendant une heure à la température ambiante, on évapore le solvant sous pression réduite, on reprend le résidu dans de l'éther éthylique, on lave avec une solution aqueuse d'acide chlorhydrique 0,2 M, à l'eau, avec une solution aqueuse de carbonate de sodium et encore à l'eau. On sèche sur du sulfate de sodium, on évapore le solvant sous pression réduite. On obtient 5 g du composé du titre.

### 1b/ 1-(2,2-diphényléthyl)-4-(3-trifluorométhylphényl)-1,2,3,6-tétrahydropyridine et son chlorhydrate

A un mélange de 0,7 g d'hydrure d'aluminium et de lithium dans 10 ml d'éther éthylique, on ajoute goutte à goutte à *25* °C une solution de 5 g (0,012 mole) du produit de l'étape précédente dans 50 ml d'éther éthylique. On agite à la température ambiante pendant une heure, on ajoute goutte à goutte 5 ml d'eau. On sépare les deux phases, on sèche la phase organique sur du sulfate de sodium et on évapore le solvant sous pression réduite. On obtient ainsi la 1-(2,2-diphényléthyl)-4-(3-trifluorométhylphényl)-1,2,3,6-tétrahydropyridine. On prépare le chlorhydrate à l'aide d'une solution d'éther éthylique saturée en acide chlorhydrique. On cristallise dans 150 ml d'acétate d'éthyle. P.f. (chlorhydrate) 207-210°C.

### EXEMPLE 2

### 1-[2,2-(4,4'-dichlorodiphényl)éthyl]-4-(3-trifluorométhylphényl)-1,2,3,6-tétrahydropyridine et son oxalate

### 2a/ α,α-(4,4'-dichlorodiphényl)acétaldéhyde

A un mélange de 5,5 g (0,025 mole ) d'iodure de triméthylsulfoxonium dans 10 ml de tétrahydrofuranne anhydre on ajoute par portions 0,75 g (0,025 mole) d'hydrure de sodium à 80% dans l'huile. On chauffe à 55°C pendant 6 heures et on y ajoute 6 g (0,025 mole) de 4,4'-dichlorobenzophénone dans 10 ml de tétrahydrofuranne anhydre. On laisse agiter le mélange à 55 °C pendant une nuit, on verse dans l'eau, on extrait à l'éther éthylique, on sèche la phase organique sur du sulfate de sodium, on évapore le solvant sous pression réduite. On dissout le résidu dans 32 ml de toluène et on y ajoute 3 ml de BF₃-Et₂O. On agite pendant 2 minutes puis on laisse reposer pendant 3 minutes. On lave deux fois avec une solution aqueuse de bicarbonate de sodium, on sèche la phase organique sur du sulfate de sodium, on évapore le solvant sous pression réduite. On obtient une huile qu'on purifie par chromatographie sur colonne de gel de silice en éluant par un mélange hexane/acétate d'éthyle = 9/1. On obtient le composé du titre.

### 2b/ 1-[2,2-(4,4'-dichlorodiphényl)éthyl]-4-(3-trifluorométhylphényl)-1,2,3,6-tétrahydropyridine et son oxalate

A la température de 0/+5°C on mélange 1,3 g (0,0045 mole) du produit de l'étape précédente, 1,2 g (0,0053 mole) de 4-(3-trifluorométhylphényl)-1,2,3,6-tétrahydropyridine, 21 ml de méthanol, 0,8 ml d'acide acétique glacial et 0,5 g d'acétate de sodium anhydre. A la même température on ajoute au mélange 0,76 g (0,0121 mole) de cyanoborohydrure de sodium, on agite pendant 1,5 heures à basse température, puis à la température ambiante pendant une nuit. On ajoute goutte à goutte 5 ml d'acide chlorhydrique concentré, on laisse agiter pendant 10 minutes, on évapore le méthanol et on reprend le résidu dans un mélange acétate d'éthyle/solution aqueuse de NH₄OH diluée. On sépare les deux phases, on sèche la phase organique sur du sulfate de sodium et on évapore le solvant sous pression réduite. On obtient une huile qu'on purifie par chromatographie sur colonne de gel de silice en éluant par un mélange hexane/acétate d'éthyle = 9/1. On obtient le composé du titre sous forme de base. On prépare l'oxalate à l'aide d'acide oxalique dans de l'isopropanol. P.f. (oxalate) 187-189°C.

### EXEMPLE 3

### 1-[2,2-(3,3'-bistrifluorométhyldiphényl)éthyl]-4-(3-trifluorométhylphényl)-1,2,3,6-tétrahydropyridine et son oxalate

### 3a/ α,α-(3,3'-bistrifluorométhyldiphényl)acétaldéhyde

En opérant comme décrit dans l'exemple 2a/, mais en utilisant la 3,3'-bistrifluorométhylbenzophénone on obtient le composé du titre.

### 3b/ 1-[2,2-(3,3'-bistrifluorométhyldiphényl)éthyl]-4-(3-trifluorométhylphényl)-1,2,3,6-tétrahydropyridine et son oxalate

En opérant comme décrit dans l'exemple 2b/, mais en utilisant le produit de l'étape précédente au lieu de l' α,α-(4,4'-dichlorodiphényl)acétaldéhyde on obtient les composés du titre. P.f. (oxalate) 194-196°C.

### EXEMPLE 4

### 1-[2,2-(4,4'-diméthoxydiphényl)éthyl]-4-(3-trifluorométhylphényl)-1,2,3,6-tétrahydropyridine et son chlorhydrate

### 4a/ α,α-(4,4'-diméthoxydiphényl)acétaldéhyde

En opérant comme décrit dans l'exemple 2a/, mais en utilisant la 4,4'-diméthoxybenzophénone on obtient le composé du titre.

### 4b/ 1-[2,2-(4,4'-diméthoxydiphényl)éthyl]-4-(3-trifluorométhylphényl)-1,2,3,6-tétrahydropyridine et son chlorhydrate

En opérant comme décrit dans l'exemple 2b/, mais en utilisant le produit de l'étape précédente au lieu de 1' α,α-(4,4'-dichlorodiphényl)acétaldéhyde on obtient les composés du titre. P.f. (chlorhydrate) 214-216°C.

### EXEMPLE 5

### 1-[2-(4-fluorophényl)-2-phényléthyl]-4-(3-trifluorométhylphényl)-1,2,3,6-tétrahydropyridine et son chlorhydrate

### 5a/ α-4-fluorophényl-α-phénylacétaldéhyde

En opérant comme décrit dans l'exemple 2a/, mais en utilisant la 4-fluorobenzophénone on obtient le composé du titre.

### 5b/ 1-[2,2-(4-fluorophényl)-2-phényléthyl]-4-(3-trifluorométhylphényl)-1,2,3,6-tétrahydropyridine et son chlorhydrate

En opérant comme décrit dans l'exemple 2b/, mais en utilisant le produit de l'étape précédente au lieu de l' α,α-(4,4'-dichlorodiphényl)acétaldéhyde on obtient les composés du titre. P.f. (chlorhydrate) 206-208°C.

### EXEMPLE 6

### 1-(3,3-diphénylpropyl)-4-(3-trifluorométhylphényl)-1,2,3,6-tétrahydropyridine et son chlorhydrate

En opérant comme décrit dans l'exemple 1b/ mais en utilisant l'acide 3,3-diphénylpropionique commercial (Aldrich, référence D21,165-6) au lieu de l'acide 2,2-diphénylacétique, on obtient les composés du titre. P.f. (chlorhydrate) 176-178°C.

### EXEMPLE 7

### 1-[2,2-(4,4'-dichlorodiphényl)éthyl]-4-(6-chloropyrid-2-yl)-1,2,3,6-tétrahydropyridine et son chlorhydrate

En opérant comme décrit dans l'exemple 2b/ mais en utilisant la 4-(6-chloropyrid-2-yl)-1,2,3,6-tétrahydropyridine au lieu de la 4-(3-trifluorométhylphényl)-1,2,3,6-tétrahydropyridine, on obtient les composés du titre. P.f. (chlorhydrate) 230-232°C.

### EXEMPLE 8

### Chlorhydrate de 1-[2-(3,4-diéthylphényl)éthyl]-4-(3-trifluorométhylphényl)-1,2,3,6-tétrahydropyridine.

### 8a/ 1-bromo-2-(3,4-diéthylphényl)éthane.

On refroidit à 0-5°C un mélange de 4,4 g (0,033 mole) de 3,4-diéthylbenzène, 50 ml de chlorure de méthylène, 8,8 g (0,044 mole) de bromure de bromoacétyle et on y ajoute 5,0 g (0,037 mole) de trichlorure d'aluminium. On agite à 0-5°C pendant une heure puis on laisse une nuit à la température ambiante. On verse dans un mélange eau/glace, on extrait au chlorure de méthylène, on sèche la phase organique sur du sulfate de sodium et on évapore le solvant sous pression réduite. On mélange 2,9 g (0,011 mole) de l'huile ainsi obtenue avec 6 ml (0,079 mole) d'acide trifluoroacétique et 6,7 ml (0,057 mole) de triéthylsilane et on chauffe à 80°C pendant 4 heures. On ajoute ensuite une solution aqueuse saturée de bicarbonate de sodium jusqu'à pH basique, on extrait à l'éther éthylique, on sèche la phase organique sur du sulfate de sodium et on évapore le solvant sous pression réduite. On purifie l'huile brute ainsi obtenue par chromatographie sur colonne de gel de silice en éluant avec du cyclohexane. On obtient le composé du titre.

### 8b/ Chlorhydrate de 1-[2-(3,4-diéthylphényl)éthyl]-4-(3-trifluorométhylphényl)-1,2,3,6-tétrahydropyridine.

On chauffe au reflux pendant 5 heures un mélange de 2,6 g (0,001 mole) de 4-(3-trifluorométhylphényl)-1,2,3,6-tétrahydropyridine, 60 ml de butanol, 4,1 g (0,025 mole) de carbonate de potassium anhydre rapé et 2,6 g (0,00113 mole) du produit de l'étape précédente. On évapore le solvant sous pression réduite, on reprend à l'acétate d'éthyle, on lave à l'eau, on sèche sur du sulfate de sodium et on évapore le solvant sous pression réduite. On prépare le chlorhydrate de l'huile ainsi obtenue par traitement avec une solution d'isopropanol saturée en acide chlorhydrique. On obtient 1,6 g du composé du titre. P.f. 220-222°C.

### EXEMPLE 9

### 1-[2-(3-méthyl-4-pentylphényl)éthyl]-4-(3-trifluorométhylphényl)-1,2,3,6-tétrahydropyridine et 1-[2-(4-méthyl-3-pentylphényl)éthyl]-4-(3-trifluorométhylphényl)-1,2,3,6-tétrahydropyridine et leurs oxalates.

### 9a/ 1-méthyl-2-pentylbenzène.

A une solution de 50 ml (0,1 mole) d'une solution de chlorure de n-butylmagnésium 2M dans le THF sous atmosphère d'azote, on ajoute goutte à goutte 4,7 g (0,035 mole) d'aldéhyde phtalique. Le mélange se chauffe spontanément à 40-45°C. On agite à la température ambiante pendant une heure, on verse dans une solution saturée de chlorure d'ammonium. On extrait à l'éther éthylique, on lave à l'eau, on sèche sur du sulfate de sodium et on évapore le solvant sous pression réduite. On purifie l'huile ainsi obtenue par chromatographie sur colonne de gel de silice en éluant par un mélange cyclohexane/acétate d'éthyle = 7/3. On isole le produit ayant le Rf le plus élevé. On obtient 2,0 g d'huile. On dissout le brut de réaction dans 25 ml d'éthanol et on y ajoute 1 ml d'acide sulfurique concentré et 0,15 g de Pd/C à 10%. On hydrogène à la température ambiante pendant 7 heures. On filtre le catalyseur, on évapore le solvant sous pression réduite et on reprend le résidu à l'acétate d'éthyle. On lave avec une solution aqueuse de bicarbonate de sodium, on sèche et on évapore le solvant sous pression réduite. On obtient 1,35 g du produit du titre.

### 9b/ 1-bromo-2-(3-méthyl-4-pentylphényl)éthane et 1-bromo-2-(4-méthyl-3-pentylphényl)éthane.

On refroidit à 0-5°C un mélange de 1,17 g (0,0054 mole) du produit de l'étape précédente, 0,62 ml (0,0072 mole) de bromure de bromoacétyle et on y ajoute 0,81 g (0,006 mole) de trichlorure d'aluminium. On agite à 0-5°C pendant une heure et ensuite 4 heures à la température ambiante. On verse dans de la glace, on sépare les deux phases, on lave la phase organique à l'eau, on la sèche et on évapore le solvant sous pression réduite. On dissout le résidu dans 2,9 ml d'acide trifluoroacétique et on y ajoute 3,1 ml (0,0267 mole) de triéthylsilane et on chauffe le mélange à 80°C pendant 5 heures. On verse dans une solution aqueuse de bicarbonate de sodium et on extrait à l'éther éthylique. On lave à l'eau, on sèche sur du sulfate de sodium. On obtient un mélange des composés du titre.

### 9c/ 1-[2-(3-méthyl-4-pentylphényl)éthyl]-4-(3-trifluorométhylphényl)-1,2,3,6-tétrahydropyridine et 1-[2-(4-méthyl-3-pentylphényl)éthyl]-4-(3-trifluorométhylphényl)-1,2,3,6-tétrahydropyridine et leurs oxalates.

On chauffe au reflux pendant 6 heures un mélange de 0,7 g (0,0031 mole) de 4-(3-trifluorométhylphényl)-1,2,3,6-tétrahydropyridine, 16 ml de butanol, 0,9 g (0,0065 mole) de carbonate de potassium anhydre rapé et le produit obtenu à l'étape précédente (0,0054 mole théorique). On évapore le solvant sous pression réduite, on reprend à l'acétate d'éthyle, on lave à l'eau, on sèche sur du sulfate de sodium et on évapore le solvant sous pression réduite. On purifie l'huile ainsi obtenue par chromatographie sur colonne de gel de silice en éluant par un mélange cyclohexane/acétate d'éthyle = 7/3. On isole deux produits ayant un Rf similaire. Le produit ayant le Rf le plus élevé correspond à la 1-[2-(3-méthyl-4-pentylphényl)éthyl]-4-(3-trifluorométhylphényl)-1,2,3,6-tétrahydropyridine. On prépare l'oxalate dans de l'acétone. On obtient 0,12 g de produit. P.f. 140-143°C. Le produit ayant le Rf le plus faible correspond à l'isomère 1-[2-(4-méthyl-3-pentylphényl)éthyl]-4-(3-trifluorométhylphényl)-1,2,3,6-tétrahydropyridine. On prépare l'oxalate dans de l'acétone. On cristallise le produit dans de l'acétone. On obtient 0,08 g de produit. P.f. 167-169°C.

### EXEMPLE 10

### Chlorhydrate de 1-[2-(3,4-diéthylphényl)éthyl]-4-(6-chloropyrid-2-yl)-1,2,3,6-tétrahydropyridine.

### 10a/ (1-benzyl-1,2,3,6-tétrahydropyrid-4-yl)tributylstannane.

On agite à la température ambiante pendant 3 heures un mélange de 15,85 g (0,0837 mole) de 1-benzyl-4-pipéridone dans 140 ml de diméthoxyéthane anhydre et 25 g (0,0837 mole) de trisilidrazine dans 140 ml de diméthoxyéthane anhydre. On évapore le solvant sous pression réduite. On reprend le résidu dans 420 ml d'hexane anhydre et on y ajoute 420 ml de tétraméthyléthylènediamine anhydre. On refroidit le mélange à -78°C et on y ajoute goutte à goutte 156 ml de n-butyl lithium (0,25 mole) (solution 1,6 M dans l'hexane). Après environ 30 minutes on laisse revenir la température jusqu'à 0°C et on agite pendant 15 minutes. On ajoute ensuite au mélange réactionnel 45 ml (0,167 mole) de chlorure de tributylstannane. Après 1 heure on ajoute, avec extrême précaution un mélange eau/glace. On extrait à l'éther éthylique, on lave la phase organique à l'eau, on sèche sur du sulfate de sodium et on évapore le solvant sous pression réduite. On obtient 70 g de produit brut qu'on purifie par chromatographie sur colonne de gel de silice en éluant avec un mélange cyclohexane/acétate d'éthyle = 95/5. On obtient le composé du titre sous forme d'huile.
¹H-RMN (CDCl₃) - δ (ppm) : 0,84 (9H; m: CH3); 1,19-1,58 (18H; m: CH₂ - chaîne); 2,31 (2H; m); 2,53 (2H; m); 3,02 (2H; m); 3,56 (2H; s: méthylène benzylique); 5,76 (1H; m*); 7,18-7,41 (5H; m: arom.)
* bandes satellites ³J_{cis}(¹H-¹¹⁷Sn) et ³J_{cis}(¹H-¹¹⁹Sn).

### 10b/ 1-benzyl-4-(6-chloropyrid-2-yl)-1,2,3,6-tétrahydropyridine.

On dissout 18,5 g (0,04 mole) du composé de l'étape précédente dans 200 ml de diméthylformamide anhydre sous atmosphère d'azote. On ajoute à la solution 11,8 g (0,08 mole) de 2,6-dichloropyridine, 0,64 g de Pd(II) (Ph₃P)₂Cl₂, 4,38 g (0,04 mole) de chlorure de tétraméthylammonium et 2,76 g (0,02 mole) de carbonate de potassium. On chauffe à 110°C pendant 6 heures puis on verse le mélange dans 100 ml d'une solution d'acide sulfurique à 5%. On extrait à l'éther éthylique, on ajoute de l'hydroxyde d'ammonium à la phase aqueuse juqu'à pH basique et on extrait à l'acétate d'éthyle. On sèche les phases organiques réunies sur du sulfate de sodium et on évapore le solvant sous pression réduite. On purifie le résidu par chromatographie sur colonne de gel de silice en éluant avec un mélange cyclohexane/acétate d'éthyle = 1/1. On obtient le composé du titre. P.f. 100-102°C.

### 10c/ Chlorhydrate de 4-(6-chloropyrid-2-yl)-1,2,3,6-tétrahydropyridine.

On refroidit à 0-5°C une solution de 7,0 g (0,024 mole) du composé de l'étape précédente dans 110 ml de dichloroéthane et on y ajoute 5,8 ml (0,054 mole) de chloroformiate de chloroéthyle. On agite pendant 5 minutes puis on chauffe au reflux pendant 1,5 heures. On évapore le solvant sous pression réduite, on reprend le résidu dans 100 ml de méthanol et on chauffe au reflux pendant 1 heure. On évapore le solvant, on reprend le résidu dans de l'isopropanol et on filtre le solide. On obtient le composé du titre qu'on cristallise dans de l'éthanol à 90%. P.f. 305-307°C.

### 10d/ Chlorhydrate de 1-[2-(3,4-diéthylphényl)éthyl]-4-(6-chloropyrid-2-yl)-1,2,3,6-tétrahydropyridine.

En opérant comme décrit dans l'exemple 8b/ mais en utilisant le produit de l'étape précédente au lieu de la 4-(3-trifluorométhylphényl)-1,2,3,6-tétrahydropyridine, on obtient le composé du titre. P.f. 234-236°C.

### EXEMPLES 11-20

En opérant comme décrit dans l'exemple 9 mais en utilisant l'halogénure de magnésium approprié, on obtient les composés suivants:
**1-[2-(3-éthyl-4-méthylphényl)éthyl]-4-(3-trifluorométhylphényl)-1,2,3,6-tétrahydropyridine - Ex. 11**
**1-[2-(4-éthyl-3-méthylphényl)éthyl]-4-(3-trifluorométhylphényl)-1,2,3,6-tétrahydropyridine - Ex. 12**
**1-[2-(3-éthyl-4-propylphényl)éthyl]-4-(3-trifluorométhylphényl)-1,2,3,6-tétrahydropyridine - Ex. 13**
**1-[2-(4-éthyl-3-propylphényl)éthyl]-4-(3-trifluorométhylphényl)-1,2,3,6-tétrahydropyridine - Ex. 14**
**1-[2-(3-butyl-4-méthylphényl)éthyl]-4-(3-trifluorométhylphényl)-1,2,3,6-tétrahydropyridine - Ex. 15**
**1-[2-(4-butyl-3-méthylphényl)éthyl]-4-(3-trifluorométhylphényl)-1,2,3,6-tétrahydropyridine - Ex. 16**
**1-[2-(3-*iso*butyl-4-méthylphényl)éthyl]-4-(3-trifluorométhylphényl)-1,2,3,6-tétrahydropyridine - Ex. 17**
**1-[2-(4-*iso*butyl-3-méthylphényl)éthyl]-4-(3-trifluorométhylphényl)-1,2,3,6-tétrahydropyridine - Ex. 18**
**1-[2-(3-*iso*butyl-4-éthylphényl)éthyl]-4-(3-trifluorométhylphényl)-1,2,3,6-tétrahydropyridine - Ex.19**
**1-[2-(4-*iso*butyl-3-éthylphényl)éthyl]-4-(3-trifluorométhylphényl)-1,2,3,6-tétrahydropyridine - Ex. 20**

### EXEMPLE 21

### 1-[2-(6-méthyl-3-biphénylyl)éthyl]-4-(3-trifluorométhylphényl)-1,2,3,6-tétrahydropyridine

En opérant comme décrit dans l'exemple 9 mais en utilisant le phényl-lithium au lieu du chlorure de n-butylmagnésium, on obtient le composé du titre.

### EXEMPLE 22

### Chlorhydrate de 1-[2-(3'-chlorobiphényl-4-yl)-2-oxoéthyl]-4-(3-trifluorométhylphényl)-1,2,3,6-tétrahydropyridine.

### 22a/ 1-bromo-2-(3'-chlorobiphényl-4-yl)éthanone.

On refroidit à 0-5°C un mélange de 5 g (0,026 mole) de 3-chlorobiphényle, 50 ml de chlorure de méthylène, 6,95 g (0,034 mole) de bromure de bromoacétyle et on y ajoute 4 g (0,030 mole) de trichlorure d'aluminium. On agite pendant 1 heure à 5°C puis 4 heures à la température ambiante. On verse dans un mélange eau/glace, on extrait au chlorure de méthylène, on lave la phase organique avec une solution IN de HCl, on sèche sur du sulfate de sodium et on évapore sous pression réduite. On obtient 4,5 g du produit du titre. P.f. 63-65°C.

### 22b/ Chlorhydrate de 1-[2-(3'-chlorobiphényl-4-yl)-2-oxoéthyl]-4-(3-trifluorométhylphényl)-1,2,3,6-tétrahydropyridine

On chauffe au reflux pendant 1 heure un mélange de 0,4 g (0,013 mole) du produit de l'étape précédente, 2,95 g (0,013 mole) de 4-(3-trifluorométhylphényl)-1,2,3,6-tétrahydropyridine, 80 ml d'éthanol et 2,32 g (0,0167 mole) de carbonate de potassium anhydre rapé. On élimine les sels par filtration, et on acidifie la solution par addition d'une solution d'éthanol saturée en acide ehlorhydrique. On concentre sous pression réduite jusqu'à environ 40 ml et on laisse une nuit à 5°C. On filtre le précipité, on le lave à l'eau et ensuite avec de l'isopropanol. On obtient 4,9 g du composé du titre. P.f. 217-220°C.

### EXEMPLE 23

### Chlorhydrate de 1-[2-(2'-chlorobiphényl-4-yl)-2-oxoéthyl]-4-(3-trifluorométhylphényl)-1,2,3,6-tétrahydropyridine.

En opérant comme décrit dans l'exemple 22 mais en utilisant le 2-chlorobiphényle au lieu du 3-chlorobiphényle, on obtient le composé du titre. P.f. 200-202°C (cristallisé dans l'isopropanol).

### EXEMPLE 24

### Chlorhydrate de 1-[2-(4'-chlorobiphényl-4-yl)-2-oxoéthyl]-4-(3-trifluorométhylphényl)-1,2,3,6-tétrahydropyridine.

En opérant comme décrit dans l'exemple 22 mais en utilisant le 4-chlorobiphényle au lieu du 3-chlorobiphényle, on obtient le composé du titre. P.f. 210-215°C.

### EXEMPLE 25

### Chlorhydrate de 1-[2-(4-isobutylphényl)-2-oxoéthyl]-4-(3-trifluorométhylphényl)-1,2,3,6-tétrahydropyridine.

En opérant comme décrit dans l'exemple 22 mais en utilisant le 4-isobutylbenzène au lieu du 3-chlorobiphényle, on obtient le composé du titre. P.f. 224-228°C (cristallisé dans l'isopropanol).

### EXEMPLE 26

### Chlorhydrate de 1-[2-(4-phénoxyphényl)-2-oxoéthyl]-4-(3-trifluorométhylphényl)-1,2,3,6-tétrahydropyridine.

En opérant comme décrit dans l'exemple 22 mais en utilisant le diphényléther au lieu du 3-chlorobiphényle, on obtient le composé du titre. P.f. 205-210°C

### EXEMPLE 27

### Chlorhydrate de 1-[2-(4-cyclohexylphényl)-2-oxoéthyl]-4-(3-trifluorométhylphényl)-1,2,3,6-tétrahydropyridine.

En opérant comme décrit dans l'exemple 22 mais en utilisant le cyclohexylbenzène au lieu du 3-chlorobiphényle, on obtient le composé du titre. P.f. 209-213°C (cristallisé dans l'isopropanol).

### EXEMPLE 28

### Chlorhydrate de 1-[2-(4'-fluorobiphényl-4-yl)-2-oxoéthyl]-4-(3-trifluorométhylphényl)-1,2,3,6-tétrahydropyridine.

En opérant comme décrit dans l'exemple 22 mais en utilisant le 4-fluorobiphényle au lieu du 3-chlorobiphényle, on obtient le composé du titre. P.f. 123-125°C (cristallisé dans l'isopropanol).

### EXEMPLE 29

### Chlorhydrate de 1-[2-(biphényl-4-yl)-2-oxoéthyl]-4-(3-trifluorométhylphényl)-1,2,3,6-tétrahydropyridine.

En opérant comme décrit dans l'exemple 22 mais en utilisant le biphényle au lieu du 3-chlorobiphényle, on obtient le composé du titre. P.f. 145-147°C (base); P.f. 240-243°C (chlorhydrate).

### EXEMPLE 30

### Chlorhydrate de 1-[2-(4-n-butylphényl)-2-oxoéthyl]-4-(3-trifluorométhylphényl)-1,2,3,6-tétrahydropyridine.

En opérant comme décrit dans l'exemple 22 mais en utilisant le 4-*n*-butylbenzène au lieu du 3-chlorobiphényle, on obtient le composé du titre, P.f. 218-221°C.

### EXEMPLE 31

### Chlorhydrate de 1-[2-(4-t-butylphényl)-2-oxoéthyl]-4-(3-trifluorométhylphényl)-1,2,3,6-tétrahydropyridine.

En opérant comme décrit dans l'exemple 22 mais en utilisant le 4-*t*-butylbenzène au lieu du 3-chlorobiphényle, on obtient le composé du titre. P.f. 97-99°C (base).

### EXEMPLE 32

### Chlorhydrate de 1-[2-(3,4-diéthylphényl)-2-oxoéthyl]-4-(3-trifluorométhylphényl)-1,2,3,6-tétrahydropyridine.

En opérant comme décrit dans l'exemple 22 mais en utilisant le 3,4-diéthylbenzène au lieu du 3-chlorobiphényle, on obtient le composé du titre. P.f. 232-234°C .

### EXEMPLE 33

### Chlorhydrate de 1-[2-(2'-trifluorométhylbiphényl-4-yl)-2-oxoéthyl]-4-(3-trifluorométhylphényl)-1,2,3,6-tétrahydropyridine.

### 33a/ 2-(4-bromophényl)-2,2-diméthoxyéthane

On chauffe au reflux pendant 3 heures un mélange de 2 g (0,01 mole) de 4-bromoacétophénone, 5,6 ml d'orthoformiate de triméthyle, 5,6 ml de méthanol et 0,67 g d'Amberlite ® IR 120. Après refroidissement, on filtre sur Célite ® et on évapore la solution filtrée. On obtient 2,4 g du produit du titre sous forme huileuse.

### 33b/ 2,2-diméthoxy-2-(2'-trifluorométhylbiphényl-4-yl)éthane

On agite à 70°C pendant 1 heure, un mélange de 4,9 g (14 mmole) du produit de l'étape précédente, 2,45 g (16 mmole) d'acide 2-trifluorométhylbenzèneboronique, 63 mg (0,28 mmole) d'acétate de palladium, 4,84 g (35 mmole) de carbonate de potassium et 4,5 g (14 mmole) de bromure de tétrabutylammonium dans 19 ml d'eau. On laisse refroidir et on extrait à l'acétate d'éthyle. On sèche la phase organique sur du sulfate de sodium, on filtre et on évapore le solvant sous pression réduite. On obtient le composé du titre sous forme huileuse.

### 33c/ 4-(2-trifluorophényl)acétophénone

A une solution de 4,6 g (0,0105 mole) du produit de l'étape précédente dans 4 ml de chlorure de méthylène, on ajoute à 0°C une solution de 4 ml d'acide trifluoroacétique et 4 ml d'eau. On agite à la température ambiante pendant 2 heures, on verse dans de l'eau, on extrait au chlorure de méthylène. On sèche la phase organique, on filtre et on évapore le solvant sous pression réduite. On purifie le brut par chromatographie sur colonne de gel de silice en éluant avec un mélange cyclohexane/acétate d'éthyle = 9/1. On obtient 1,97 g du produit du titre.

### 33d/ α-bromo-4-(2-trifluorométhylphényl)acétophénone

A une solution de 1,97 g (7,5 mmole) du produit de l'étape précédente dans 5,4 ml de méthanol, on ajoute goutte à goutte à la température de 0°C, 0,38 ml (7,5 mmole) de brome. On agite à la température ambiante pendant 3 heures, on évapore le solvant, on reprend le résidu dans de l'eau et on extrait à l'acétate d'éthyle. On sèche la phase organique sur du sulfate de sodium, on filtre et on évapore le solvant sous pression réduite. On obtient le produit du titre sous forme huileuse.

### 33e/ Chlorhydrate de 1-[2-(2'-trifluorométhylbiphényl-4-yl)-2-oxoéthyl]-4-(3-trifluorométhylphényl)-1,2,3,6-tétrahydropyridine

On chauffe au reflux pendant 1 heure un mélange de 0,74 g (0,0028 mole) de 4-(3-trifluorométhylphényl)-1,2,3,6-tétrahydropyridine, 14 ml d'éthanol et 1,27 g (0,0092 mole) de carbonate de potassium anhydre rapé. On y ajoute une solution de 1,2 g (0,0035 mole) de l'huile de l'étape précédente dans 3 ml d'éthanol et on laisse au reflux pendant 30 minutes. On élimine les sels par filtration, et on acidifie la solution par addition d'une solution aqueuse d'acide chlorhydrique 1 N. On évapore le solvant sous pression réduite, on extrait au chloroforme, on sèche la phase organique sur du sulfate de sodium, on filtre et on évapore le solvant sous pression réduite. On libère la base à l'aide d'une solution d'ammoniaque concentrée, on extrait à l'acétate d'éthyle, et on purifie le produit par chromatographie sur colonne de gel de silice en éluant avec un mélange cyclohexane/acétate d'éthyle = 8/2. On obtient le composé du titre. On prépare le chlorhydrate à l'aide d'une solution d'isopropanol saturée en acide chlorhydrique. P.f. 195-197°C.

### EXEMPLE 34

### Chlorhydrate de 1-[2-(3'-trifluorométhylbiphényl-4-yl)-2-oxoéthyl]-4-(3-trifluorométhylphényl)-1,2,3,6-tétrahydropyridine.

En opérant comme décrit dans l'exemple 33 mais en utilisant l'acide 3-trifluorométhylbenzèneboronique au lieu de l'acide 2-trifluorométhylbenzèneboronique dans l'étape 33b/, on obtient le composé du titre. P.f. 232-234°C.

### EXEMPLE 35

### Chlorhydrate de 1-[2-(4'-trifluorométhylbiphényl-4-yl)-2-oxoéthyl]-4-(3-trifluorométhylphényl)-1,2,3,6-tétrahydropyridine.

En opérant comme décrit dans l'exemple 33 mais en utilisant l'acide 4-trifluorométhylbenzèneboronique au lieu de l'acide 2-trifluorométhylbenzèneboronique dans l'étape 33b/, on obtient le composé du titre. P.f. 245-247°C.

### EXEMPLE 36

Un mélange de 12,5 g de 2-(2-bromoéthyl)naphtalène, 14 g de chlorhydrate de 4-(3-trifluorométhylphényl)-1,2,3,6-tétrahydropyridine, 4,34 g d'hydroxyde de sodium, 135 ml d'eau et 95 ml d'éthanol 95% est chauffé pendant 5 heures au reflux, puis on laisse refroidir le mélange réactionnel pendant une nuit à la température ambiante. On refroidit le mélange en dessous de 25°C, puis on filtre, on lave à l'eau le produit ainsi isolé et on le sèche sous vide à 50 °C. On obtient ainsi la 1-[2-(2-naphtyl)éthyl]-4-(3-trifluorométhylphényl)-1,2,3,6-tétrahydropyridine base avec un rendement de 90 % calculé sur le chlorhydrate de 4-(3-trifluorométhylphényl)-1,2,3,6-tétrahydropyridine de départ.

### EXEMPLE 37

Un mélange de 19,5 g de chlorhydrate de 1-[2-(2-naphtyl)éthyl]-4-(3-trifluorométhylphényl)-1,2,3,6-tétrahydropyridine brut, 95 ml d'éthanol absolu et 4,65 ml d'acide chlorhydrique à 37% est chauffé au reflux sous agitation jusqu'à dissolution complète, puis on laisse refroidir, toujours sous agitation. Lorsque les premiers cristaux commencent à se former (vers 63°C), l'agitation est arrêtée et le mélange réactionnel est maintenu à 0-5°C pendant une nuit. Après filtration, le produit est réempâté deux fois dans 30 ml d'éthanol absolu, puis seché pendant une nuit à 40°C sous vide.
Dans ces conditions, on a obtenu 12,8 g de Forme I du chlorhydrate de 1-[2-(2-naphtyl)éthyl]-4-(3-trifluorométhylphényl)-1,2,3,6-tétrahydropyridine.
A l'analyse calorimétrique différentielle, la Forme I obtenue dans cette préparation a présenté
· une température de transition solide-solide de 148-149°C
· une enthalpie de transition de 26,4 J/g.

### EXEMPLE 38

Dans un réacteur calorimétrique METTLER RC1 muni d'un agitateur à palettes ("impeller") de 8 cm de diamètre, un mélange de 70 g de chlorhydrate de 1-[2-(2-naphtyl)éthyl]-4-(3-trifluorométhylphényl)-1,2,3,6-tétrahydropyridine brut et de 1 1 d'éthanol absolu est chauffé au reflux jusqu'à dissolution complète du produit. La solution ainsi obtenue est refroidie avec une vitesse de refroidissement de 80°C par heure et une vitesse d'agitation de 500 tr/minute jusqu'à 10°C. Le précipité ainsi obtenu est filtré et seché une nuit à 45°C sous vide. Dans ces conditions, on a obtenu la Forme II du chlorhydrate de 1-[2-(2-naphtyl)éthyl]-4-(3-trifluorométhylphényl)-1,2,3,6-tétrahydropyridine.
A l'analyse calorimétrique différentielle, la Forme II obtenue dans cette préparation a présenté
· une température de transition solide-solide de 153-155°C
· une enthalpie de transition de 24,1 J/g.

### EXEMPLE 39

Un mélange de 2 g de chlorhydrate de 1-[2-(2-naphtyl)éthyl]-4-(3-trifluorométhylphényl)-1,2,3,6-tétrahydropyridine et de 50 ml de diméthylsulfoxyde est chauffé au reflux jusqu'à dissolution complète, on laisse refroidir le mélange pendant une nuit, puis on récupère le produit cristallin et on le sèche sous vide à 45° C pendant une nuit.
Dans ces conditions, on a obtenu la Forme III du chlorhydrate de 1-[2-(2-naphtyl)éthyl]-4-(3-trifluorométhylphényl)-1,2,3,6-tétrahydropyridine.
A l'analyse calorimétrique différentielle, la Forme III obtenue dans cette préparation a présenté
· une température de transition solide-solide de 141-142°C
· une enthalpie de transition de 17,6 J/g.

### EXEMPLE 40

Un mélange de 100 g de chlorhydrate de 1-[2-(2-naphtyl)éthyl]-4-(3-trifluorométhylphényl)-1,2,3,6-tétrahydropyridine dans 1 l d'un mélange éthanol/eau 90/10 est chauffé au reflux sous agitation jusqu'à dissolution complète du produit. La solution ainsi obtenue est refroidie de la température de reflux à 5°C sous agitation impeller à 400 tr/minute à une vitesse de refroidissement de 10°C/heure. Le produit cristallin ainsi obtenu est filtré et seché à 45°C sous vide pendant une nuit.
Dans ces conditions, on a obtenu le chlorhydrate de 1-[2-(2-naphtyl)éthyl]-4-(3-trifluorométhylphényl)-1,2,3,6-tétrahydropyridine sous forme de mélange Forme I/Forme III, dans un rapport 65,7/34,3.
A l'analyse calorimétrique différentielle, la Forme I/III obtenue dans cette préparation présente un thermogramme qui montre uniquement les deux pics caractéristiques correspondant aux Formes I et III.

### EXEMPLE 41

Une solution de 3 g de chlorhydrate de 1-[2-(2-naphtyl)éthyl]-4-(3-trifluorométhylphényl)-1,2,3,6-tétrahydropyridine dans 300 ml d'éthanol est atomisée dans un appareil "mini Spray Dryer Buchi" selon le principe de l'atomisation par buse en courant parallèle, en réglant le débit de la pompe, l'aspiration, le chauffage et le flux de courant de façon à avoir une température d'entrée de 172°C, une température de sortie de 107°C et une dépression de 40 mbar. Dans ces conditions, on obtient un produit monopic évasé en DSC avec le maximum à 145°C. Les particules obtenues sont sphériques et la population très homogène ne dépasse pas 5 micromètres de taille moyenne.

### EXEMPLE 42

On introduit dans la chambre de micronisation (diamètre 200 mm) d'un microniseur ALPINE 200 AS 24 kg de chlorhydrate de 1-[2-(2-naphtyl)éthyl]-4-(3-trifluorométhylphényl)-1,2,3,6-tétrahydropyridine- Forme I/III, décrit dans l'Exemple 40, à une vitesse de 25 kg/heure et à une pression de travail de 6,5 bar et on recupère le produit ainsi micronisé dans une manche filtrante. On obtient ainsi un produit micronisé ayant une distribution particulaire selon laquelle la totalité des particules a une taille inférieure à 20 micromètres et 85% des particules ont une taille inférieure à 10 micromètres.
L'analyse calorimétrique différentielle du produit micronisé ainsi obtenu montre que les températures de transition ne sont pas affectées par la micronisation. Lesdites transitions sont du type solide-solide. Le composé se dégrade avant la fusion, qui commence à 250 °C.

### EXEMPLE 43

Composition pharmaceutique contenant, en tant que principe actif, le chlorhydrate de 1-[2-(2-naphtyl)éthyl]-4-(3-trifluorométhylphényl)-1,2,3,6-tétrahydropyridine Forme I/III (micronisée) selon l'Exemple 42 ci-dessus:

| | |
|---|---|
| Principe actif | 2,192 mg |
| Amidon de maïs | 141,208 mg |
| Cellulose microcristalline | 26,000 mg |
| Silice colloïdale anhydre | 0,200 mg |
| Stéarate de magnésium | 0,400 mg |

Le principe actif est tamisé à 0,2 mm, puis prémélangé avec les excipients. Ce mélange est tamisé à 0,315 mm, remélangé, puis à nouveau tamisé à 0,315 mm. Après un dernier mélange, on introduit la composition dans des gélules de gélatine n° 3, à raison de 170 mg de composition contenant une quantité de chlorhydrate de 1-[2-(2-naphtyl)éthyl]-4-(3-trifluorométhylphényl)-1,2,3,6-tétrahydropyridine - Forme I/III correspondant à 2 mg de 1-[2-(2-naphtyl)éthyl]-4-(3-trifluorométhylphényl)-1,2,3,6-tétrahydropyridine base.

### EXEMPLE 44

Des boîtes contenant des cellules sont préparées comme dans la PREPARATION 1. On mesure les taux de TGF-β1 comme décrit dans la PREPARATION 2. On mesure les taux de TGF-β1 activé dans les milieux extracellulaires en présence du SR 57746A après 1, 3, 14, 24 et 48 heures et 7 jours de contact avec les cellules, en comparaison avec les témoins (0,2 % de sérum foetal de veau et 20% de sérum foetal de veau) par la méthode décrite dans la PREPARATION 2. Le SR 57746 induit une augmentation significative des taux de TGF-β1 activé dans les milieux extracellulaires après 14 heures de contact avec les cellules.

### EXEMPLE 45

On prépare des boîtes contenant des cellules comme dans la PREPARATION 1 et on induit l'apoptose comme dans la PREPARATION 4 selon le méthode a). On mesure les effets anti-apoptotiques du SR 57746 et des composés A, B, C, D, E, F après 1, 3, 14, 24, 48 heures et 7 jours de contact avec les cellules en comparaison avec les témoins (0,2 % de sérum foetal de veau et 20% de sérum foetal de veau) par la méthode décrite dans la PREPARATION 4.
Les composés testés inhibent de manière significative l'apoptose induite par déprivation de sérum dès 24 heures de contact avec les cellules et pendant 7 jours au moins.

### EXEMPLE 46

Des boîtes contenant des cellules sont préparées comme dans la PREPARATION 1. On induit l'apoptose selon la méthode b) de la PREPARATION 4. Les taux d'apoptose sont mesurés après 24 heures de contact avec les cellules par la méthode décrite dans la PREPARATION 4 ; les même témoins que dans la PREPARATION 4 sont utilisés. Le SR 57746 ainsi que les composés A, B, C, D, E, F inhibent de façon significative l'effet pro-apoptotique du NGF.

### EXEMPLE 47

On prépare des boîtes contenant des cellules comme décrit dans la PREPARATION 1. On induit l'apoptose selon la méthode c) de la PREPARATION 4. Les taux d'apoptose sont mesurés après 24 heures de contact avec les cellules par la méthode décrite dans la PREPARATION 4 ; les même témoins que dans la PREPARATION 4 sont utilisés. Le SR 57746 et les composés A, B, C, D, E, F inhibent de façon significative l'effet pro-apoptotique de la vincristine.

### EXEMPLE 48

### Chlorhydrate de 1-1-[2-(6,7-méthylènedioxynapht-2-yl)-éthyl]-4-[3-trifluorométhylphényl)-1,2,3,6-tétrahydropyridine.

On agite à température ambiante 1,1 g (0,0048 mole) d'acide 2-(6,7-diméthoxynapht-2-yl)acétique, 20 ml de chlorure de méthylène, 2 ml (0,0144 mole) de triéthylamine, 1,35 g (0,0048 mole) de 4-(3-trifluorométhylphényl)-4-pipéridinol et 2,15 g (0,0048 mole) de BOP. On lave avec une solution de HCI 1 N, ensuite avec une solution saturée en NaHCO₃ et ensuite avec de l'eau. On sèche sur du sulfate de sodium et on évapore le solvant sous pression réduite. On dissout 1,5 g de l'huile ainsi obtenue dans 18 ml de THF. On chauffe au reflux et on y ajoute, goutte à goutte, une solution de 0,97 ml (0,0102 mole) de diméthylsulfure de borane dans 12 ml de THF. On chauffe le mélange au reflux pendant 4 heures, on refroidit à 0°C et on ajoute 15 ml de méthanol. On chauffe encore au reflux pendant 30 min. et après on évapore sous pression réduite. On reprend le résidu avec de l'acétate d'éthyle, on lave à l'eau, on sèche sur du sulfate de sodium et on évapore sous pression réduite. On dissout l'huile obtenue dans 22 ml d'acide acétique glacial, on y ajoute 1,2 ml d'acide sulfurique concentré et on chauffe à 60°C pendant 4 heures. On verse dans un mélange glace/NaOH et on extrait à l'acétate d'éthyle. On prépare le chlorhydrate à l'aide d'isopropanol saturé en HCl en obtenant le composé du titre P.f. 277-280°C.

### EXEMPLE 49

### 49a/Chlorhydrate de 4-(2-trifluorométhylphényl)-1,2,3,6-tétrahydropyridine.

On chauffe à 100°C pendant deux heures un mélange de 2 g (0.0071 mole) de 4-(2-trifluorométhylphényl)-4-pipéridinol (préparé à partir de benzylpipéridone, 2-bromo-1-trifluorométhylbenzène et magnesium et successive hydrogénation) 12 ml d'acide acétique glacial et 3 ml d'acide sulfurique concentré. On verse dans un mélange glace/NaOH et on extrait au chlorure de méthylène. On sèche la phase organique et on évapore sous pression réduite. On prépare le chlorhydrate à l'aide d'isopropanol saturé en acide chlorhydrique. P.f. 213-215°C.

### 49b/Chlorhydrate de 1-[2-(biphényl-4-yl)éthyl]-4-(2-trifluorométhylphényl)-1,2,3,6-tétrahydropyridine

En opérant comme décrit dans l'exemple 8b mais en utilisant le produit de l'étape précédente au lieu de la 4-(3-trifluorométhylphényl)- 1,2,3,6-tétrahydropyridine et le 1-bromo-2-biphénylyléthane au lieu du produit de l'étape 8a, on obtient le composé du titre. P.f. 273-275°C.

### EXEMPLE 50

### Chlorhydrate de 1-[2-(4-cyclohexènylphényl)éthyl]-4-(3-trifluorométhylphényl)- 1,2,3,6-tétrahydropyridine

On agite, à 70°C pendant 3 heures, un mélange de 1 g (0,005 mole) de 2-(4-bromophényl)éthanol, 0,7 g (0,0055 mole) d'acide 1-cyclohexèneboronique, 25 mg d'acétate de palladium, 1,73 g (0,0012 mole) de carbonate de potassium et 1,61 g (0,005 mole) de bromure de tétrabutylammonium dans 7 ml d'eau. On laisse refroidir et on extrait à l'acétate d'éthyle. On sèche la phase organique sur du sulfate de sodium, on filtre et on évapore le solvant sous pression réduite. On purifie le brut de réaction par chromatographie sur colonne de gel de silice, en éluant avec un mélange cyclohexane /acétate d'éthyle = 7/3. On obtient le composé du titre sous forme huileuse. On refroidit à 0,5°C une solution de 1,35 g (6,67 mmole) du produit de l'étape précédente, 0,93 ml (6,67 mole) de chlorure de mésyle dans 0,5 ml de chlorure de méthylène. On agite à 0°C pendant 30 minutes et ensuite pendant 1 nuit à température ambiante. On verse le mélange dans de l'eau et on extrait au chlorure de méthylène. On sèche la phase organique et évapore le solvant sous pression réduite. On reprend le résidu dans 8 ml d'isopropanol, on y ajoute 0,64 ml (4,6 mmole) de triéthylamine et 0,45 g (1,7 mmole) de 4-(3-trifluorométhylphényl)-1,2,3,6-tétrahydropyridine. On chauffe le mélange au reflux pendant 4 heures, on évapore le solvant et on lave à l'eau. On extrait au chlorure de méthylène, on sèche la phase organique et on évapore le solvant sous pression réduite. On purifie le résidu par chromatographie sur colonne de gel de silice en éluant avec un mélange cyclohexane/acétate d'éthyle = 8/2. On prépare le chlorhydrate à l'aide d'isopropanol saturé en HCl. On obtient le composé du titre. P.f. 244-245°C.

## Revendications

1. Utilisation d'un composé de formule (I): 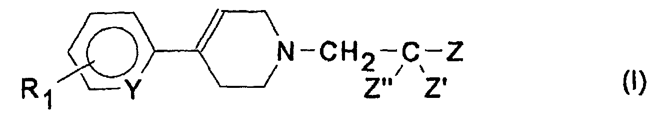 dans laquelle:
- R1 représente un halogène ou un groupe CF₃, (C₁-C₄)alkyle ou (C₁-C₄)alcoxy;
- Y représente un atome d'azote ou un groupe CH;
- Z' et Z" représentent chacun l'hydrogène ou un groupe (C₁-C₃) alkyle, ou bien l'un représente l'hydrogène et l'autre un groupe hydroxy, ou encore les deux, ensemble, représentent un groupe oxo;
- Z représente
◆ un radical phényle;
◆ un radical phényle monosubstitué par un substituant X, X étant
a) un groupe (C₁-C₆)alkyle; (C₁-C₆)alcoxy; (C₃-C₇)carboxyalkyle; (C₁-C₄)alcoxycarbonyl(C₁-C₆)alkyle; (C₃-C₇)carboxyalcoxy ou (C₁-C₄)-alcoxycarbonyl(C₁-C₆)alcoxy;
b) un groupe choisi parmi un (C₃-C₇)cycloalkyle, (C₃-C₇)cycloalkyloxy, (C₃-C₇)cycloalkylméthyle, (C₃-C₇)cycloalkylamino et cyclohexényle, ledit groupe pouvant être substitué par un halogène, hydroxy, (C₁-C₄)alcoxy, carboxy, (C₁-C₄)alcoxycarbonyle, amino, mono- ou di-(C₁-C₄)alkylamino;
c) un groupe choisi parmi un phényle, phénoxy, phénylamino, N-(C₁-C₃)alkylphénylamino, phénylméthyle, phényléthyle, phénylcarbonyle, phénylthio, phénylsulfonyle, phénylsulfinyle ou styryle, ledit groupe pouvant être mono- ou polysubstitué sur le groupe phényle par un halogène, CF₃, (C₁-C₄)alkyle, (C₁-C₄)alcoxy, cyano, amino, mono- ou di-(C₁-C₄)alkylamino, (C₁-C₄)acylamino, carboxy, (C₁-C₄)alcoxycarbonyle, aminocarbonyle, mono- ou di-(C₁-C₄)alkylaminocarbonyle, amino(C₁-C₄)alkyle, hydroxy(C₁-C₄)alkyle ou halogéno(C₁-C₄)alkyle;
◆ un radical phényle disubstitué par un substituant R₂, R₂ étant un halogène ou un groupe hydroxy, méthyle, éthyle, (C₃-C₆)alkyle, (C₁-C₄)alcoxy ou trifluorométhyle et par un substituant X, X étant tel que défini ci-dessus;
◆ un radical 1-naphtyle ou 2-naphtyle;
◆ un radical 1-naphtyle ou 2-naphtyle, substitué dans les positions 5, 6, 7 et/ou 8 par un ou deux groupes hydroxyle, un ou deux groupes (C₁-C₄)alcoxy ou un groupe 6, 7-méthylénedioxy;
- ou bien Z" est l'hydrogène et Z et Z' représentent, chacun indépendamment, un groupe phényle non substitué ou mono-di ou trisubstitué;
ou d'un de ses sels et solvates pharmaceutiquement acceptables, pour la préparation de compositions pharmaceutiques aptes à augmenter les taux circulants et cellulaires et extracellulaires de TGF-β_{1,}, utiles pour le traitement des maladies choisies parmi les maladies à prions, le glaucome; l'ostéoporose et les fractures osseuses.

2. Utilisation selon la revendication 1, **caractérisée en ce que**, dans ledit composé de formule (I), Y est CH et R₁ est *o*- ou *m*-CF₃.

3. Utilisation selon la revendication 2, **caractérisée en ce que** Z' et Z" sont l'hydrogène.

4. Utilisation selon la revendication 2, **caractérisée en ce que** Z' et Z" forment ensemble un groupe oxo et Z est le 4-biphényle.

5. Utilisation selon la revendication 3, **caractérisée en ce que** Z représente un groupe 2-naphtyle, 6,7-diméthoxy-2-naphtyle ou 6,7-méthylènedioxy-2-naphtyle.

6. Utilisation selon la revendication 3, **caractérisée en ce que** Z représente un radical phényle monosubstitué par un substituant X, X étant tel que défini dans la revendication 1.

7. Utilisation selon la revendication 3, **caractérisée en ce que** Z représente un radical phényle monosubstitué par un groupe X', X' étant un phényle, non substitué ou substitué par 1 à 3 halogène, 1 à 3 CF₃, 1 à 3 (C₁-C₄)alkyle, 1 à 3 (C₁-C₄)alcoxy, 1 à 3 cyano, 1 à 3 amino, 1 à 3 mono- ou di-(C₁-C₄)alkylamino, 1 à 3 (C₁-C₄)acylamino, 1 à 3 carboxy, 1 à 3 (C₁-C₄)alcoxycarbonyle, 1 à 3 aminocarbonyle, 1 à 3 mono- ou di-(C₁-C₄)alkylaminocarbonyle, 1 à 3 amino(C₁-C₄)alkyle, 1 à 3 hydroxy(C₁-C₄)alkyle ou 1 à 3 halogéno(C₁-C₄)alkyle; ou bien un radical phényle disubstitué par un substituant R₂, R₂ étant tel que défini dans la revendication 1 et par un substituant X', X' étant tel que défini ci-dessus.

8. Utilisation selon la revendication 3, **caractérisée en ce que** Z est un groupe phényle disubstitué dans les positions 3 et 4 par un groupe méthyle, éthyle ou (C₃-C₆) alkyle.

9. Utilisation selon la revendication 2, **caractérisée en ce que** Z" est l'hydrogène et Z et Z', identiques, représentent chacun un groupe phényle ; un groupe phényle substitué en position 2, 3 ou 4 par un atome de fluor, de chlore ou par un groupe méthyle, éthyle, *n*-propyle, *i*-propyle, *n*-butyle, *i*-butyle, *s*-butyle, *t*-butyle trifluorométhyle, cyano, méthoxy, méthylthio, méthylsulfonyle, éthoxy, éthylthio, éthylsulfonyle, (C₁-C₃)alcoxycarbonyle ou di(C₁-C₃)alkylaminocarbonyle; un groupe phényle disubstitué dans les positions 2, 4; 3, 4; 3, 5 ou 2, 6 par un atome de chlore, de fluor, ou par un groupe méthyle, éthyle, trifluorométhyle, cyano ou méthoxy; ou un groupe phényle trisubstitué dans les positions 3, 4, 5; 2, 4, 5 ou 2, 4, 6 par un atome de chlore, de fluor, ou par un groupe méthyle, éthyle, trifluorométhyle, cyano ou méthoxy.

10. Utilisation selon la revendication 3, **caractérisée en ce que** le composé de formule (I) est le chlorhydrate de 1-[2-(2-naphtyl)éthyl]-4-(3-trifluorométhyl-phényl)-1,2,3,6-tétrahydropyridine.

11. Utilisation selon la revendication 10, **caractérisée en ce que** le chlorhydrate de 1-[2-(2-naphtyl)éthyl]-4-(3-trifluorométhylphényl)-1,2,3,6-tétrahydropyridine est sous forme atomisée ou micronisée.

12. Utilisation selon la revendication 10, **caractérisée en ce que** le chlorhydrate de 1-[2-(2-naphtyl)éthyl]-4-(3-trifluorométhylphényl)-1,2,3,6-tétrahydropyridine est un melange micronisé des formes cristallines I et III dans le rapport d'environ 66/34.

13. Utilisation selon la revendication 1, **caractérisée en ce que** les compositions pharmaceutiques sont indiquées pour le traitement des maladies à prions.

14. Un composé choisi parmi la 1-[2-(6,7-méthylènedioxynapht-2-yl)éthyl]-4-(3-trifluorométhylphényl)-1,2,3,6-tétrahydropyridine, la 1-[2-(4-cyclohexénylphényl)éthyl]-4-(3-trifluorométhylphényl)-1,2,3,6-tétrahydropyridine et la 1-[2-(biphényl-4-yl)éthyl]-4-(2-trifluorométhylphényl)-1,2,3,6-tétrahydropyridine et leurs sels et solvates pharmaceutiquement acceptables.

## Patentansprüche

1. Verwendung einer Verbindung der Formel (I): 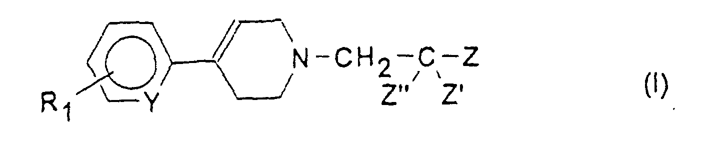 in der:
- R₁ ein Halogenatom oder eine CF₃-Gruppe, (C₁-C₄)-Alkylgruppe oder (C₁-C₄)-Alkoxygruppe darstellt;
- Y ein Stickstoffatom oder eine Gruppe CH bedeutet;
- Z' und Z" jeweils Wasserstoff oder eine (C₁-C₃)-Alkylgruppe bedeuten oder eine dieser Gruppen Wasserstoff und die andere eine Hydroxygruppe oder beide gemeinsam eine Oxogruppe darstellen;
- Z:
◆ einen Phenylrest;
◆ einen durch einen Substituenten X monosubstituierten Phenylrest, worin X
a) eine (C₁-C₆)-Alkylgruppe; (C₁-C₆)-Alkoxygruppe; (C₃-C₇)-Carboxyalkylgruppe; (C₁-C₄)-Alkoxycarbonyl-(C₁-C₆)-alkylgruppe; (C₃-C₇)-Carboxyalkoxygruppe oder (C₁-C₄)-Alkoxycarbonyl-(C₁-C₆)-alkoxygruppe;
b) eine Gruppe ausgewählt aus (C₃-C₇)-Cycloalkyl, (C₃-C₇)-Cycloalkyloxy, (C₃-C₇)-Cycloalkylmethyl, (C₃-C₇)-Cycloalkylamino und Cyclohexenyl, wobei diese Gruppe durch ein Halogen, Hydroxy, (C₁-C₄)-Alkoxy, Carboxy, (C₁-C₄)-Alkoxycarbonyl, Amino, Mono- oder Di-(C₁-C₄)-alkylamino substituiert sein kann;
c) eine Gruppe ausgewählt aus Phenyl, Phenoxy, Phenylamino, N-(C₁-C₃)-Alkylphenylamino, Phenylmethyl, Phenylethyl, Phenylcarbonyl, Phenylthio, Phenylsulfonyl, Phenylsulfinyl oder Styryl, wobei die Gruppe an der Phenylgruppe durch ein Halogen, CF₃- (C₁-C₄)-Alkyl, (C₁-C₄)-Alkoxy, Cyano, Amino, Mono- oder Di-(C₁-C₄)-alkylamino, (C₁-C₄)-Acylamino, Carboxy, (C₁-C₄)-Alkoxycarbonyl, Aminocarbonyl, Mono- oder Di-(C₁-C₄)-alkylaminocarbonyl, Amino- (C₁-C₄)-alkyl, Hydroxy- (C₁-C₄)-alkyl oder Halogen- (C₁-C₄)-alkyl mono- oder polysubstituiert sein kann;
darstellt:
◆ einen Phenylrest, der disubstituiert ist durch einen Substituenten R₂, worin R₂ ein Halogen oder eine Hydroxygruppe, Methylgruppe, Ethylgruppe, (C₃-C₆)-Alkylgruppe, eine (C₁-C₄)-Alkoxygruppe oder eine Trifluormethylgruppe bedeutet, und durch einen Substituenten X, worin X die oben angegebenen Bedeutungen besitzt;
◆ eine 1-Naphthyl- oder 2-Naphthylgruppe;
◆ eine 1-Naphthyl- oder 2-Naphthylgruppe, die in den Positionen 5, 6, 7 und/oder 8 durch eine oder zwei Hydroxylgruppen, eine oder zwei (C₁-C₄)-Alkoxygruppen oder eine 6,7-Methylendioxygruppe substituiert ist,
darstellt;
- oder Z" Wasserstoff und Z und Z' jeweils unabhängig voneinander eine nichtsubstituierte oder mono-, di- oder trisubstituierte Phenylgruppe bedeuten;
oder eines ihrer pharmazeutisch annehmbaren Salze und Solvate, für die Herstellung von pharmazeutischen Zubereitungen, die dazu geeignet sind, die zellulär und extrazellulär zirkulierenden Spiegel von TGF-b₁ zu erhöhen, die nützlich sind für die Behandlung von Erkrankungen ausgewählt aus Prionen-Erkrankungen, Glaukom, Osteoporose und Knochenbrüchen.

2. Verwendung nach Anspruch 1, **dadurch gekennzeichnet, daß** in der Verbindung der Formel (I) Y CH und R₁ *o*- oder *m*-CF₃ bedeuten.

3. Verwendung nach Anspruch 2, **dadurch gekennzeichnet, daß** Z' und Z" Wasserstoff bedeuten.

4. Verwendung nach Anspruch 2, **dadurch gekennzeichnet, daß** Z' und Z" gemeinsam eine Oxogruppe bilden und Z 4-Biphenyl bedeutet.

5. Verwendung nach Anspruch 3, **dadurch gekennzeichnet, daß** Z eine 2-Naphthyl-, 6,7-Dimethoxy-2-naphthyl- oder 6,7-Methylendioxy-2-naphthylgruppe bedeutet.

6. Verwendung nach Anspruch 3, **dadurch gekennzeichnet, daß** Z einen Phenylrest bedeutet, der durch einen Substituenten X monosubstituiert ist, wobei X die in Anspruch 1 angegebenen Bedeutungen besitzt.

7. Verwendung nach Anspruch 3, **dadurch gekennzeichnet, daß** Z einen Phenylrest, der durch eine Gruppe X' monosubstituiert ist, worin X' Phenyl, das durch eine Gruppe X' monosubstituiert ist, worin X' nichtsubstituiertes Phenyl oder Phenyl substituiert durch 1 bis 3 Halogenatome, 1 bis 3 CF₃, 1 bis 3 (C₁-C₄)-Alkyl, 1,bis 3 (C₁-C₄)-Alkoxy, 1 bis 3 Cyano, 1 bis 3 Amino, 1 bis 3 Mono- oder Di-(C₁-C₄)-alkylamino, 1 bis 3 (C₁-C₄)-Acylamino, 1 bis 3 Carboxy, 1 bis 3 (C₁-C₄)-Alkoxycarbonyl, 1 bis 3 Aminocarbonyl, 1 bis 3 Mono- oder Di- (C₁-C₄)-Alkylamlnocarbonyl, 1 bis 3 Amino- (C₁-C₄)-alkyl, 1 bis 3 Hydroxy- (C₁-C₄)-alkyl oder 1 bis 3 Halogen- (C₁-C₄)-alkyl darstellt; oder einen Phenylrest, der disubstituiert ist durch einen Substituenten R₂, worin R₂ die in Anspruch 1 angegebenen Bedeutungen besitzt, und durch einen Substituenten X', worin X' die oben angegebenen Bedeutungen besitzt, darstellt.

8. Verwendung nach Anspruch 3, **dadurch gekennzeichnet, daß** Z eine Phenylgruppe ist, die in den Positionen 3 und 4 durch eine Methyl-, Ethyl- oder (C₃-C₆)-Alkylgruppe disubstituiert ist.

9. Verwendung nach Anspruch 2, **dadurch gekennzeichnet, daß** Z" Wasserstoff und Z und Z', die identisch sind, jeweils eine Phenylgruppe; eine in der 2-, 3-oder 4-Position durch ein Fluoratom, Chloratom oder eine Methyl-, Ethyl-, *n*-Propyl-, Isopropyl-, *n*-Butyl-, Isobutyl-, *sec*.-Butyl-, *tert*.-Butyl-, Trifluormethyl-, Cyano-, Methoxy-, Methylthio-, Methylsulfonyl-, Ethoxy-, Ethylthio-, Ethylsulfonyl-, (C₁-C₃)-Alkoxycarbonyl- oder Di- (C₁-C₃)-alkylaminocarbonylgruppe substituierte Phenylgruppe; eine Phenylgruppe, die in den Positionen 2, 4; 3, 4; 3, 5 oder 2, 6 durch ein Chloratom, ein Fluoratom oder eine Methyl-, Ethyl-, Trifluormethyl-, Cyano- oder Methoxygruppe disubstituiert ist; oder eine Phenylgruppe, die in den Positionen 3, 4, 5; 2, 4, 5 oder 2, 4, 6 durch ein Chloratom, Fluoratom oder eine Methyl-, Ethyl-, Trifluormethyl-, Cyano- oder Methoxygruppe trisubstituiert ist. bedeuten.

10. Verwendung nach Anspruch 3, **dadurch gekennzeichnet, daß** die Verbindung der Formel (I) 1-[2-(2-Naphthyl)-ethyl]-4-(3-trifluormethylphenyl)-1,2,3,6-tetrahydropyridin-Hydrochlorid ist.

11. Verwendung nach Anspruch 10, **dadurch gekennzeichnet, daß** das 1-[2-(2-Naphthyl)-ethyl]-4-(3-trifluormethylphenyl)-1,2,3,6-tetrahydropyridin-Hydrochlorid in atomisierter oder mikronisierter Form verwendet wird.

12. Verwendung nach Anspruch 10, **dadurch gekennzeichnet, daß** das 1-[2-(2-Naphthyl)-ethyll-4-(3-trifluormethylphenyl)-1,2,3,6-tetrahydropyridin-Hydrochlorid in Form einer mikronisierten Mischung der kristallinen Formen I und III in einem Verhältnis von etwa 66/34 vorliegt.

13. Verwendung nach Anspruch 1, **dadurch gekennzeichnet, daß** die pharmazeutischen Zubereitungen für die Behandlung von Prionen-Erkrankungen indiziert sind.

14. Verbindung ausgewählt aus 1-[2-(6,7-Methylendioxynaphth-2-yl)-ethyll-4-(3-trifluormethylphenyl)-1,2,3,6-tetrahydropyridin, 1-[2-(4-Cyclohexenylphenyl)-ethyl]-4-(3-trifluormethylphenyl)-1,2,3,6-tetrahydropyridin und 1-[2-(Biphenyl-4-yl)-ethyl]-4-(2-trifluormethylphenyl)-1,2,3,6-tetrahydropyridin und deren pharmazeutisch annehmbaren Salzen und Solvaten.

## Claims

1. Use of a compound of formula (I): 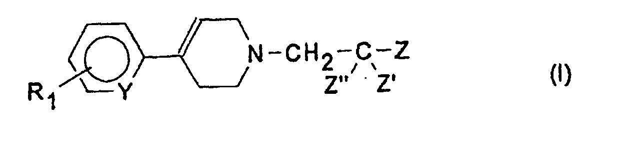 in which:
- R₁ represents a halogen or a group CF₃, (C₁-C₄)alkyl or (C₁-C₄)alkoxy;
- Y represents a nitrogen atom or a CH group;
- Z' and Z" each represent hydrogen or a (C₁-C₃)alkyl group, or one represents hydrogen and the other represents a hydroxyl group, or alternatively both, together, represent an oxo group;
- Z represents
◆ a phenyl radical;
◆ a phenyl radical monosubstituted with a substituent X, X being
a) a group (C₁-C₆)alkyl; (C₁-C₆)alkoxy; (C₃-C₇)carboxyalkyl; (C₁-C₄)alkoxycarbonyl(C₁-C₆)alkyl; (C₃-C₇)carboxyalkoxy or (C₁-C₄)alkoxycarbonyl(C₁-C₆)alkoxy;
b) a group chosen from a (C₃-C₇)cycloalkyl, (C₃-C₇)cycloalkyloxy, (C₃-C₇) cycloalkylmethyl, (C₃-C₇)cycloalkylamino and cyclohexenyl, the said group possibly being substituted with a halogen, hydroxyl, (C₁-C₄)alkoxy, carboxyl, (C₁-C₄)alkoxycarbonyl, amino or mono- or di(C₁-C₄)alkylamino;
c) a group chosen from a phenyl, phenoxy, phenylamino, N-(C₁-C₃)alkylphenylamino, phenylmethyl, phenylethyl, phenylcarbonyl, phenylthio, phenylsulphonyl, phenylsulphinyl or styryl, the said group possibly being mono- or polysubstituted on the phenyl group with a halogen, CF₃, (C₁-C₄)alkyl, (C₁-C₄)alkoxy, cyano, amino, mono- or di(C₁-C₄)alkylamino, (C₁-C₄)acylamino, carboxyl, (C₁-C₄)alkoxycarbonyl, aminocarbonyl, mono- or di(C₁-C₄)alkylaminocarbonyl, amino(C₁-C₄)alkyl, hydroxy(C₁-C₄)alkyl or halo(C₁-C₄)alkyl;
◆ a phenyl radical disubstituted with a substituent R₂, R₂ being a halogen or a hydroxyl, methyl, ethyl, (C₃-C₆)alkyl, (C₁-C₄)alkoxy or trifluoromethyl group and with a substituent X, X being as defined above;
◆ a 1-naphthyl or 2-naphthyl radical;
◆ a 1-naphthyl or 2-naphthyl radical substituted in positions 5, 6, 7 and/or 8 with one or two hydroxyl groups, one or two (C₁-C₄)alkoxy groups or a 6,7-methylenedioxy group;
- or alternatively Z" is hydrogen and Z and Z' each independently represent an unsubstituted or mono-, di- or trisubstituted phenyl group;
or one of the pharmaceutically acceptable salts and solvates thereof, for the preparation of pharmaceutical compositions capable of increasing the cellular and extracellular circulating levels of TGF-β₁, which are useful for treating diseases chosen from prion diseases, glaucoma, osteoporosis and bone fractures.

2. Use according to Claim 1, **characterized in that**, in the said compound of formula (I), Y is CH and R₁ is *o*- or *m*-CF₃.

3. Use according to Claim 2, **characterized in that** Z' and Z" are hydrogen.

4. Use according to Claim 2, **characterized in that** Z' and Z" together form an oxo group and Z is 4-biphenyl.

5. Use according to Claim 3, **characterized in that** Z represents a 2-naphthyl, 6,7-dimethoxy-2-naphthyl or 6,7-methylenedioxy-2-naphthyl group.

6. Use according to Claim 3, **characterized in that** Z represents a phenyl radical monosubstituted with a substituent X, X being as defined in Claim 1.

7. Use according to Claim 3, **characterized in that** Z represents a phenyl radical monosubstituted with a group X', X' being a phenyl, which is unsubstituted or substituted with 1 to 3 halogen, 1 to 3 CF₃, 1 to 3 (C₁-C₄)alkyl, 1 to 3 (C₁-C₄)alkoxy, 1 to 3 cyano, 1 to 3 amino, 1 to 3 mono- or di(C₁-C₄)alkylamino, 1 to 3 (C₁-C₄)acylamino, 1 to 3 carboxyl, 1 to 3 (C₁-C₄)alkoxycarbonyl, 1 to 3 aminocarbonyl, 1 to 3 mono- or di(C₁-C₄)alkylaminocarbonyl, 1 to 3 amino(C₁-C₄)alkyl, 1 to 3 hydroxy(C₁-C₄)alkyl or 1 to 3 halo(C₁-C₄)alkyl; or alternatively a phenyl radical disubstituted with a substituent R₂, R₂ being as defined in Claim 1 and with a substituent X', X' being as defined above.

8. Use according to Claim 3, **characterized in that** Z is a phenyl group disubstituted in positions 3 and 4 with a methyl, ethyl or (C₃-C₆)alkyl group.

9. Use according to Claim 2, **characterized in that** Z" is hydrogen and Z and Z', which are identical, each represent a phenyl group; a phenyl group substituted in position 2, 3 or 4 with a fluorine or chlorine atom or with a methyl, ethyl, *n*-propyl, *i*-propyl, *n*-butyl, *i*-butyl, *s*-butyl, *t*-butyl, trifluoromethyl, cyano, methoxy, methylthio, methylsulphonyl, ethoxy, ethylthio, ethylsulphonyl, (C₁-C₃)alkoxycarbonyl or di(C₁-C₃)alkylaminocarbonyl group; a phenyl group disubstituted in positions 2,4; 3,4; 3,5 or 2,6 with a chlorine or fluorine atom or with a methyl, ethyl, trifluoromethyl, cyano or methoxy group; or a phenyl group trisubstituted in positions 3,4,5; 2,4,5 or 2,4,6 with a chlorine or fluorine atom or with a methyl, ethyl, trifluoromethyl, cyano or methoxy group.

10. Use according to Claim 3, **characterized in that** the compound of formula (I) is 1-[2-(2-naphthyl)ethyl]-4-(3-trifluoromethylphenyl)-1,2,3,6-tetrahydropyridine hydrochloride.

11. Use according to Claim 10, **characterized in that** the 1-[2-(2-naphthyl)ethyl]-4-(3-trifluoromethylphenyl)-1,2,3,6-tetrahydropyridine hydrochloride is in atomized or micronized form.

12. Use according to Claim 10, **characterized in that** the 1-[2-(2-naphthyl)ethyl]-4-(3-trifluoromethylphenyl)-1,2,3,6-tetrahydropyridine hydrochloride is a micronized mixture of the I and III crystal forms in a ratio of about 66/34.

13. Use according to Claim 1, **characterized in that** the pharmaceutical compositions are indicated for the treatment of prion diseases.

14. Compound chosen from 1-[2-(6,7-methylenedioxynaphth-2-yl)ethyl]-4-(3-trifluoromethylphenyl)-1,2,3,6-tetrahydropyridine, 1-[2-(4-cyclohexenylphenyl)ethyl]-4-(3-trifluoromethylphenyl)-1,2,3,6-tetrahydropyridine and 1-[2-(biphenyl-4-yl)ethyl]-4-(2-trifluoromethylphenyl)-1,2,3,6-tetrahydropyridine, and the pharmaceutically acceptable salts and solvates thereof.
